# EUROPEAN PATENT APPLICATION

(11) **EP 3 756 680 A1**
(43) Date of publication of application: **30.12.2020**
(21) Application number: 19382537.9
(22) Date of filing: 26.06.2019
(51) Int. Cl.: A61K 38/17, C07K 14/47

(54) **INTERMEDIATE FILAMENT-DERIVED PEPTIDES AND THEIR USES**

(71) Applicant: Universitat de Lleida, 25003 Lleida (ES); Institut de Recerca Biomèdica de Lleida Fundació Dr Pifarré, 25198 Lérida (ES); Fundació Institut D'investigació En Ciències De La Salut Germans Trias i Pujol, 08916 Badalona (ES); Fundació Institut de Recerca Hospital Universitari Vall d'Hebron, 08035 Barcelona (ES)
(72) Inventor: VERDAGUER AUTONELL, Joan, E- 25003 Lleida (ES); EGIA MENDIKUTE, Leire, E-25198 Lleida (ES); CORRAL PUJOL, Marta, E- 25003 Lleida (ES); MORA GIRAL, Concepció, E- 25003 Lleida (ES); ROSELL MASES, Estela, E-25198 Lleida (ES); VIVES PI, Marta, E-08916 Badalona, Barcelona (ES); PUJOL AUTONELL, Irma, E-08916 Badalona, Barcelona (ES); BARQUINERO MÁÑEZ, Jordi, E-08035 Barcelona (ES)
(74) Representative: ABG Intellectual Property Law, S.L.

(57) **Abstract**

The present invention relates to peptides derived from known intermediate filaments which are capable of inducing cell death in metazoan cells, and/or stimulating pro-inflammatory cytokine secretion. The peptides consist of a first region of "n" amino acids, wherein "n" is 0 to 41 amino acids; a second region of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and a third region of "m" amino acids, wherein "m" is 0 to 41 amino acids. The peptides of the invention have a minimum length of 9 amino acids and a maximum length of 50 amino acids. These peptides may be useful as new adjuvants in vaccines, either alone or in combination with other therapies; as well as chemotherapeutic agents, either alone or in combination with other drugs or therapies.

## Description

### FIELD OF THE INVENTION

The present invention relates to peptides derived from known intermediate filaments and their uses as new adjuvants in vaccines and as chemotherapeutic agents against cancer, either alone or in combination with other drugs or therapies.

### BACKGROUND ART

Most commercial adjuvants are microorganism-derived products called PAMPs (Pathogen Associated Molecular Pattern). PAMPs are usually capable of inducing a good response, but they may also induce excessively high local inflammation and systemic adverse effects. For this reason, they are reserved for veterinary and experimental use vaccines. In addition, some salts such as aluminium hydroxide and some emulsions such as MF59 may also act as adjuvants. Aluminium hydroxide is very well tolerated, being the most commonly used adjuvant in vaccines intended for human consumption. MF59 is a squalene preparation specially designed for use as an adjuvant in influenza vaccines. The main problem with these latter adjuvants for use in human vaccines is that they usually induce a good response in terms of stimulating the production of blocking antibodies. However, they are not that efficient in stimulating Th1 CD4+ and cytotoxic CD8+ T-lymphocyte responses, which results in a lack of efficiency to act against intracellular infections.

### SUMMARY OF THE INVENTION

The present invention relates to the surprising finding that peptides derived from known intermediate filaments are capable of inducing apoptosis, pyroptosis and/or necroptosis in all tested types of eukaryotic cells. In addition, in leukocytes, they stimulate pro-inflammatory cytokine secretion, and may thus be useful as new adjuvants in vaccines, either alone or in combination with other therapies; as well as chemotherapeutic agents, either alone or in combination with other drugs or therapies.

Thus, in a first aspect, the invention relates to a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, with the proviso that the peptide sequence is not any of the following:
(i) YQELMNVKLALDIEIATYRR (SEQ ID NO: 2);
(ii) YQDLLNVKMALDIEIATYRR (SEQ ID NO: 3);
(iii) YQDLLNVKLALDIEIATYRR (SEQ ID NO: 4);
(iv) YQDLLNVKMALDVEIATYRR (SEQ ID NO: 5);
(v) YQELMNVKLALDIEIATYRK (SEQ ID NO: 6);
(vi) YQDLLNVKMALDIEIATYRK (SEQ ID NO: 7);
(vii) YQDLLNVKLALDIEIATYRK (SEQ ID NO: 8);
(viii) YQDLLNVKMALDVEIATYRK (SEQ ID NO: 9);
(ix) DYQELMNVKLALDVEIATYR (SEQ ID NO: 10);
(x) VKIALEVEIATY (SEQ ID NO: 11);
(xi) IKSRLEQEIATYRSLLEGQEDHYNNLSASKVL (SEQ ID NO: 12);
(xii) LMDIKSRLEQEIATY (SEQ ID NO: 13).

In another aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, for use in medicine, with the proviso that the peptide sequence is not VKIALEVEIATY (SEQ ID NO: 11).

In a further aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, for use as an adjuvant.

In another further aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, for use in the treatment of cancer.

In another aspect, the invention relates to a vaccine comprising, as an adjuvant, the peptide according to the present invention.

In a final aspect, the invention relates to a use of a peptide according to the present invention as a cell death-inducing agent.

### BRIEF DESCRIPTION OF THE FIGURES

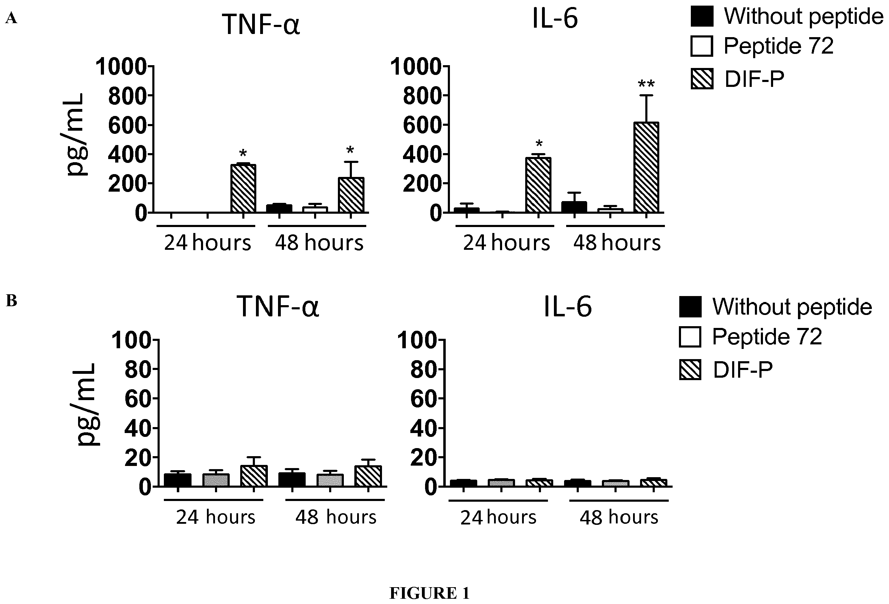

**Figure 1** - Cytokine concentration from mouse splenocytes cultured with peptides. Results of the analysis of the supernatants of cells incubated for 24h and 48h with peptides 72 and DIF-P (n = 4); (A) splenocytes from NOD.RAG-2-/- mice; (B) splenocytes from NOD mice.

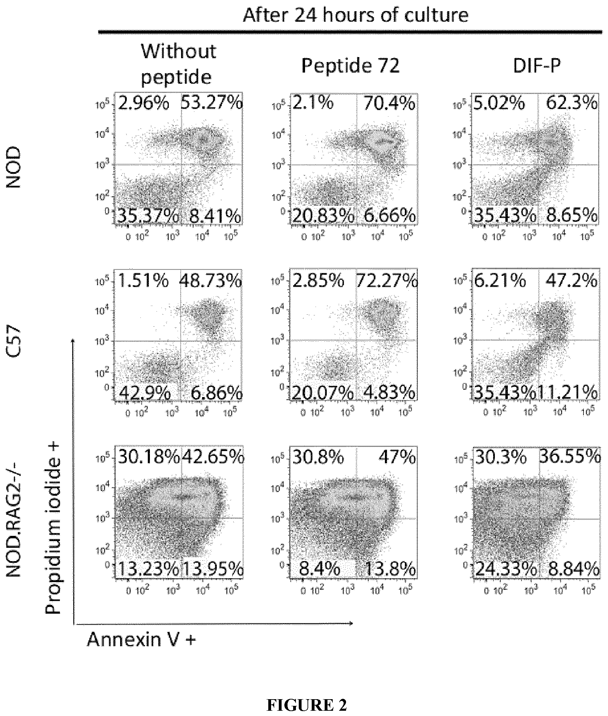

**Figure 2** - Apoptosis assay results from NOD, NOD.RAG2-/- and C57B1/6 incubated with peptides. Representative images of dot plots obtained after 24 hours of incubation of splenocytes from NOD, NOD.RAG2-/- and C57B1/6 with peptides from Peripherin (n=3).

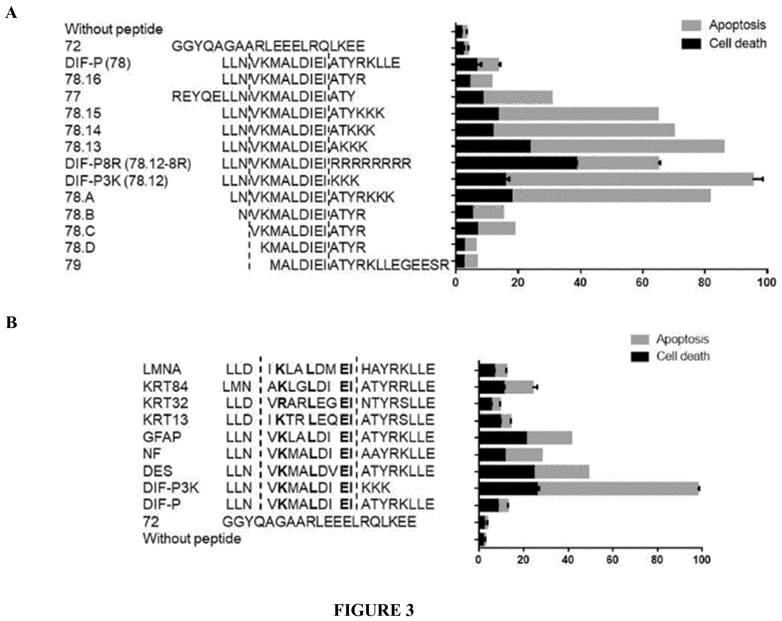

**Figure 3** - Cell death and apoptosis of NALM6 cells after culture with different variants of DIF. Annexin positive (marker of cellular apoptosis), and Annexin V and propidium iodide double positive (markers of cell death) NALM-6 cells, 24 h after culture in presence of different peptide fragments DIF-P, and DIF variants. A) 72, 77, DIF-P (also known as peptide 78), and 79 stand for different peptides derived from the Peripherin intermediate filament. To determine the active core sequence, amino acids from the native sequence of DIF-P (peptide 78) were removed or replaced by lysine AAs in the carboxyterminal region. From DIF-P3K (78.12 peptide) through peptide 78.16, and from peptide 78.A through 78.D represent different variants of DIF-P (DIF-P variants). The replacement of lysines was carried out for matters of the different peptide synthesis requirements. Polar amino acid (R or K) sequences act as cell-penetrating peptides that help cell peptide entry. In DIF-P8R (also known as a 78-12.8R peptide), the native 78 peptide carboxyterminal region after the core sequence, was replaced by a tail of arginines in order to increase peptide penetrance into cell cytoplasm. B) DIF-P (peptide 78), NF, GFAP, DES, LMNA, KRT84, KRT32 and KRT13 are different peptides coming from the core consensus sequence of Peripherin/Vimentin, Neurofilament (heavy, medium and light), Glial Fibrillar Acidic Protein, Desmin, Lamin A, Keratin-84, Keratin-32 and Keratin-13 intermediate filaments respectively. 72 is an irrelevant peptide derived from Peripherin, here used as a negative control. NALM-6 is a human B lymphocyte leukemia cell line.

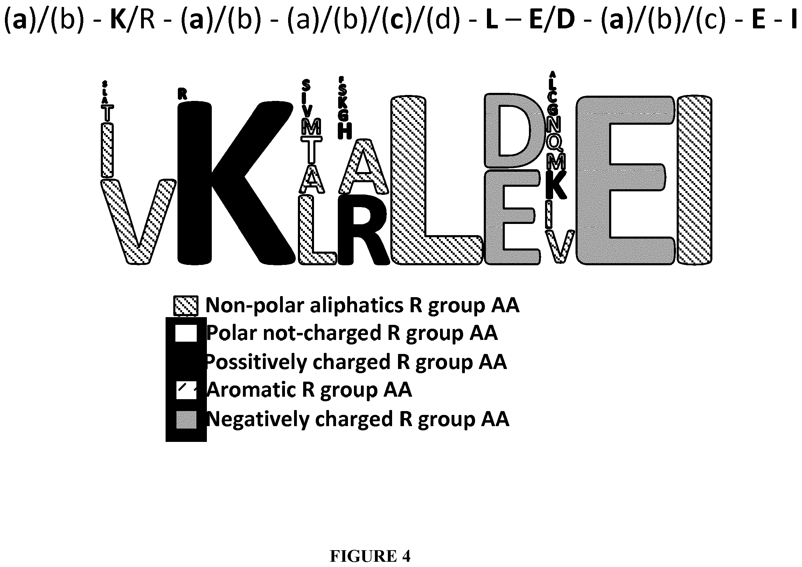

**Figure 4** - Summary sequence (DIF sequence) including all possible changes found in all intermediate filaments. The letter size represents the probability of that specific amino acid in the sequence; other probable amino acid variations are also represented per each position.

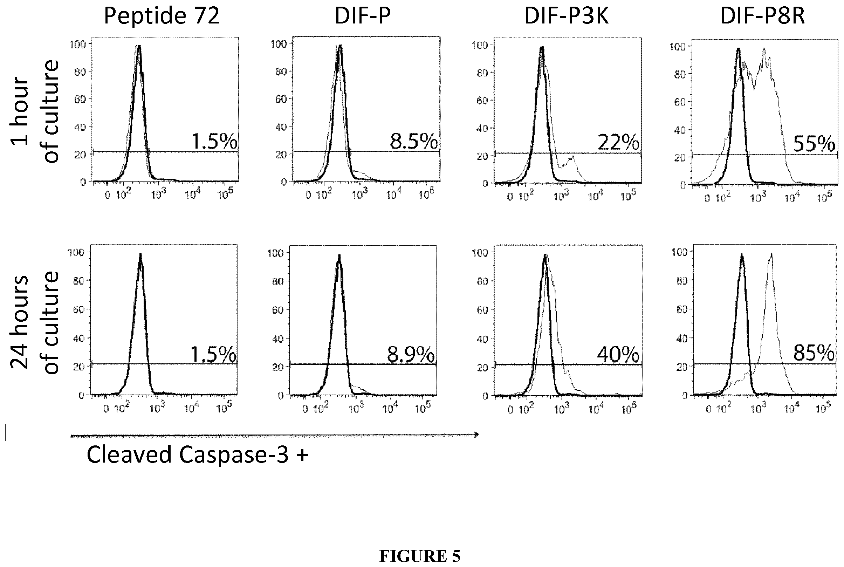

**Figure 5** - Representative images of the expression of cleaved Caspase-3 in NALM-6 cells cultured with different peptides. NALM-6 cells were cultured with peptide 72, or DIF-P, or DIF-P3K, or DIF-P8R or without stimulus for 1 hour or 24 hours and intracellular staining was performed to analyse the expression of cleaved caspase-3. The line in bold is equivalent to the expression of the control cells without peptide, and clearer curves are equivalent to cells incubated with different peptides (n = 3).

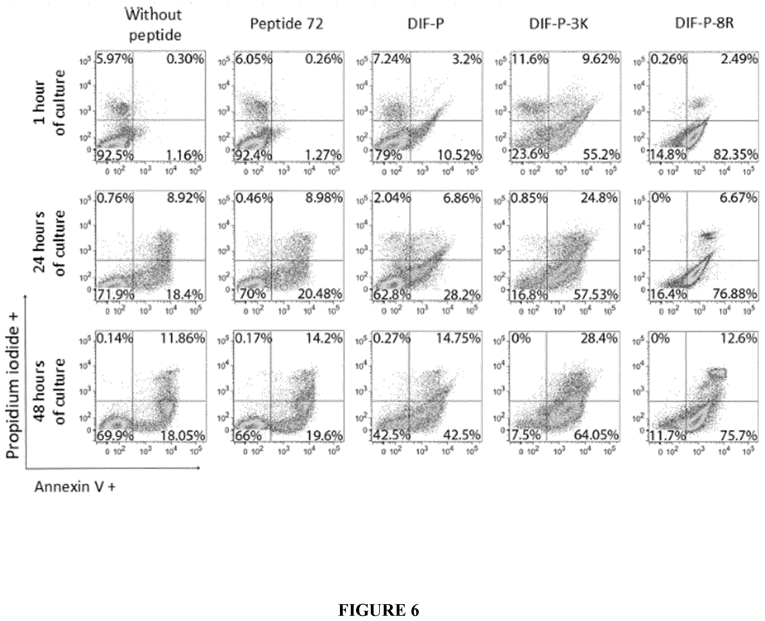

**Figure 6** - Representative dot plots of apoptosis assay of human PBMC cells after culturing them with different peptides. Annexin V and PI positive cells were analysed after culturing 4x10⁵ cells for 1 hour, 24 hours or 48 hours with peptide 72, or DIF-P, or DIF-P3K, or DIF-P8R or without stimulus (n = 4).

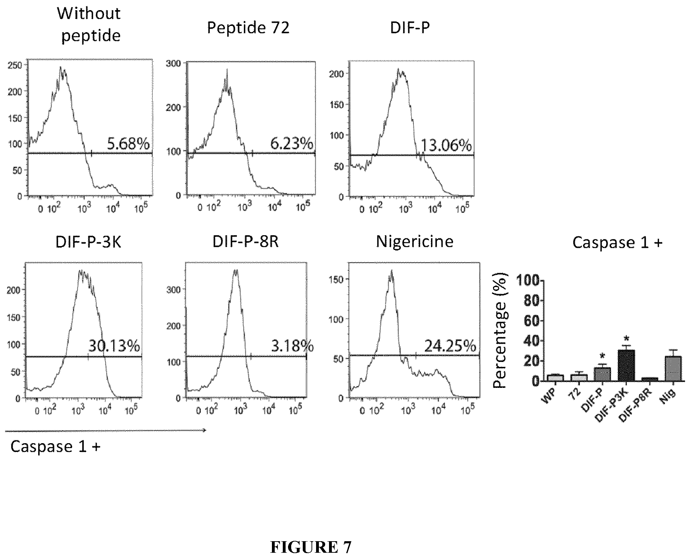

**Figure 7** - Representative histograms of activated (cleaved) caspase-1 positive cells from human PBMC cultured with different peptides. 4x10⁵ PBMC cells were cultured with peptide 72, or DIF-P, or DIF-P3K, or DIF-P8R, or Nigericin (as a positive control) or without stimulus for an hour and activated caspase-1 was measured with 660-YVAD-FMK (n=4). Positive cell percentage is resumed on the right of the figure (*=p<0.05).

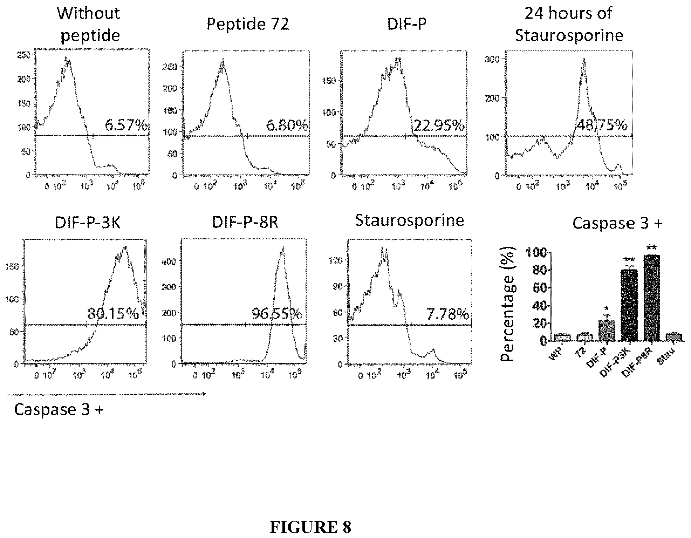

**Figure 8** - Representative histograms of activated (cleaved) caspase-3 positive cells from human PBMC cultured with different peptides. 4x10⁵ PBMC cells were cultured with peptide 72, or DIF-P, or DIF-P3K, or DIF-P8R, or staurosporine (as a positive control) or without stimulus for an hour and activated caspase-3 was measured with FAM-DEVD-FMK (n=4). Positive cell percentage is resumed on the right of the figure (*=p<0.05, **=p<0.01).

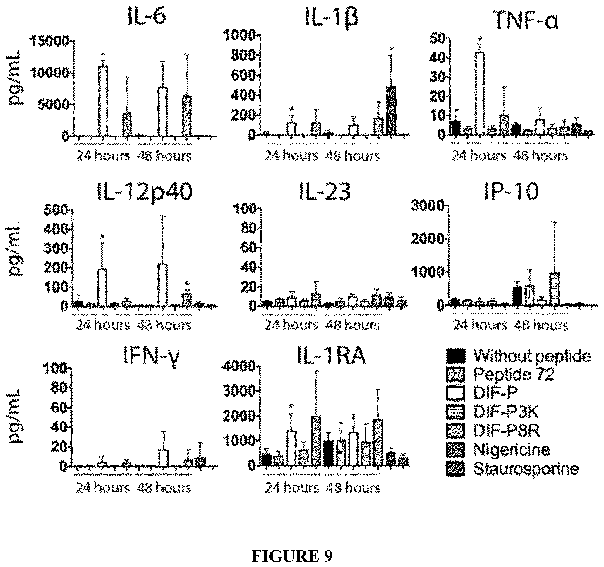

**Figure 9** - Concentrations of different cytokines produced by human PBMC cells cultured with peptides. 4x10⁵ cells were incubated per well with peptide 72, or DIF-P, or DIF-P3K, or DIF-P8R, or Staurosporine, or Nigericine or without stimulus for 24 hours or 48 hours before collecting the supernatant. In the case of Staurosporine and Nigericine the production of cytokines was only analysed after 24 hours (* = p < 0.05).

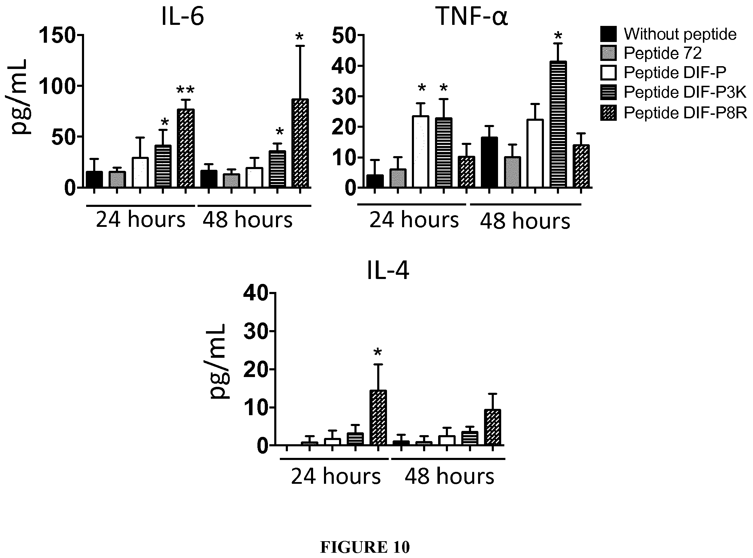

**Figure 10** - Concentrations of different cytokines produced by NOD.RAG2-/- splenocytes cultured with peptides. 4x10⁵ cells were incubated per well with peptide 72, or DIF-P, or DIF-P3K, or DIF-P8R, or without stimulus for 24 hours or 48 hours before collecting the supernatant (* = p < 0.05, **=p<0.01).

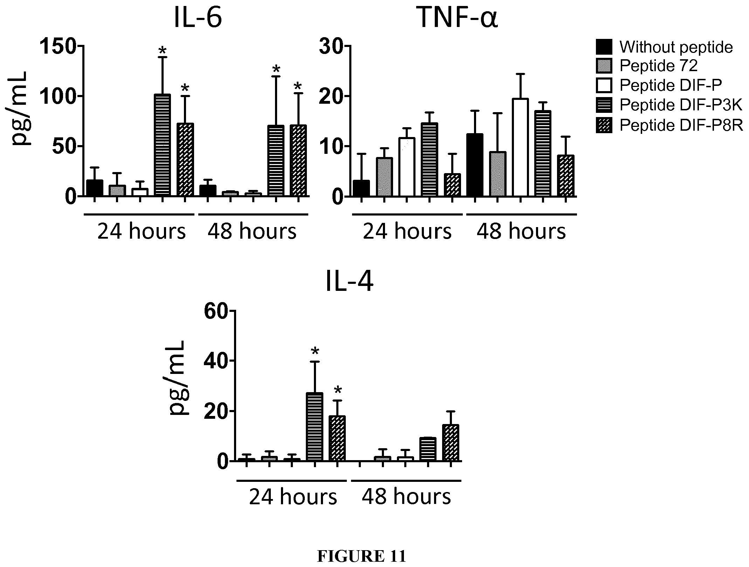

**Figure 11** - Concentrations of different cytokines produced by NOD.RAG2-/- cells from bone marrow cultured with peptides. 4x10⁵ cells were incubated per well with peptide 72, or DIF-P, or DIF-P3K, or DIF-P8R, or without stimulus for 24 hours or 48 hours before collecting the supernatant (* = p < 0.05).

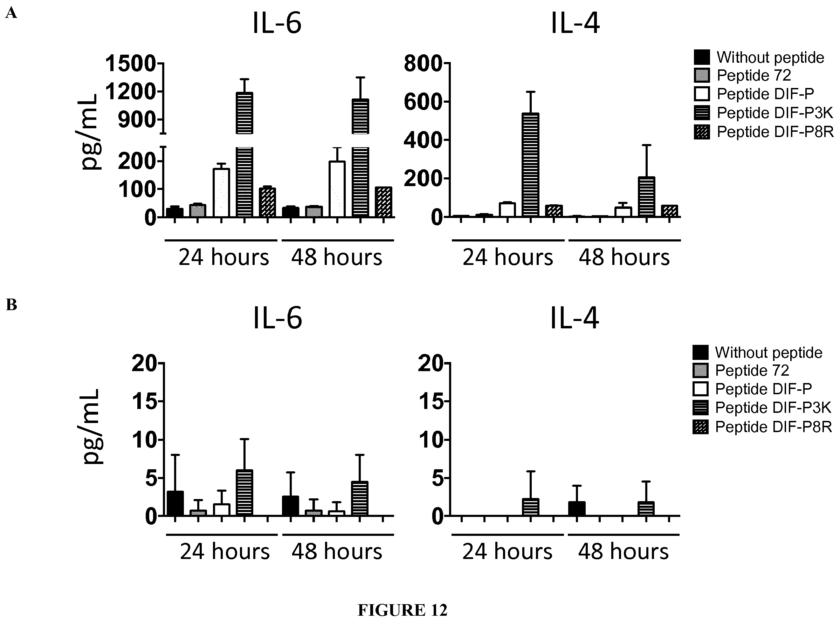

**Figure 12** - Concentrations of different cytokines produced by purified cells from NOD bone marrow cultured with peptides. 4x10⁵ cells were incubated per well with peptide 72, or DIF-P, or DIF-P3K, or DIF-P8R, or without stimulus for 24 hours or 48 hours before collecting the supernatant. Cells are (A) monocytes and (B) neutrophils.

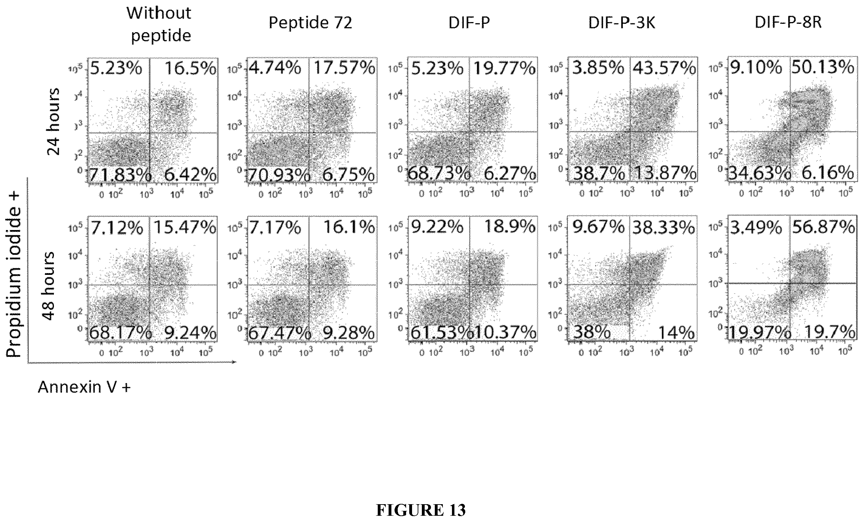

**Figure 13** - Representative dot plots of apoptosis assay of NOD.RAG2-/- bone marrow cells after culturing them with different peptides. Annexin V and PI positive cells were analysed after culturing 4x10⁵ cells for 24 hours or 48 hours with peptide 72, or DIF-P, or DIF-P3K, or without stimulus (n = 4).

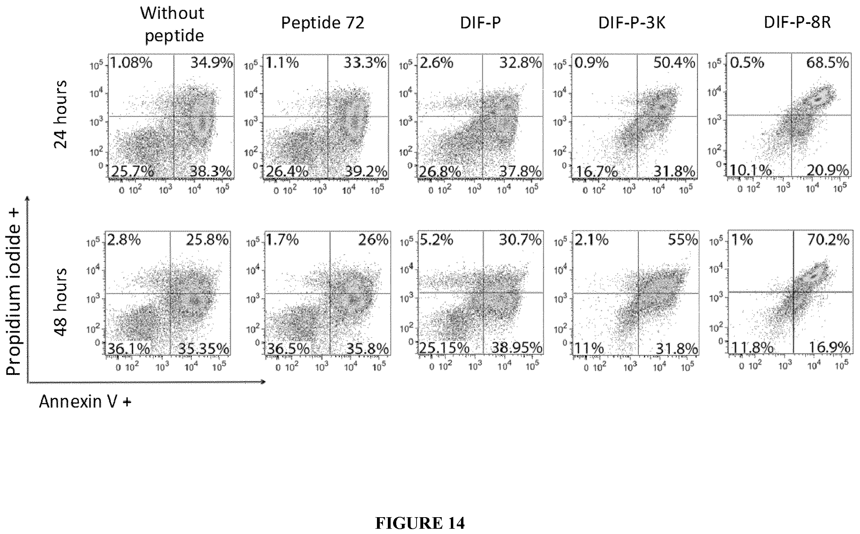

**Figure 14** - Representative dot plots of apoptosis assay of purified monocytes from NOD bone marrow after culturing them with different peptides. Annexin V and PI positive cells were analysed after culturing 4x10⁵ monocytes for 24 or 48 hours with peptide 72, or DIF-P, or DIF-P3K, or without stimulus (n = 4).

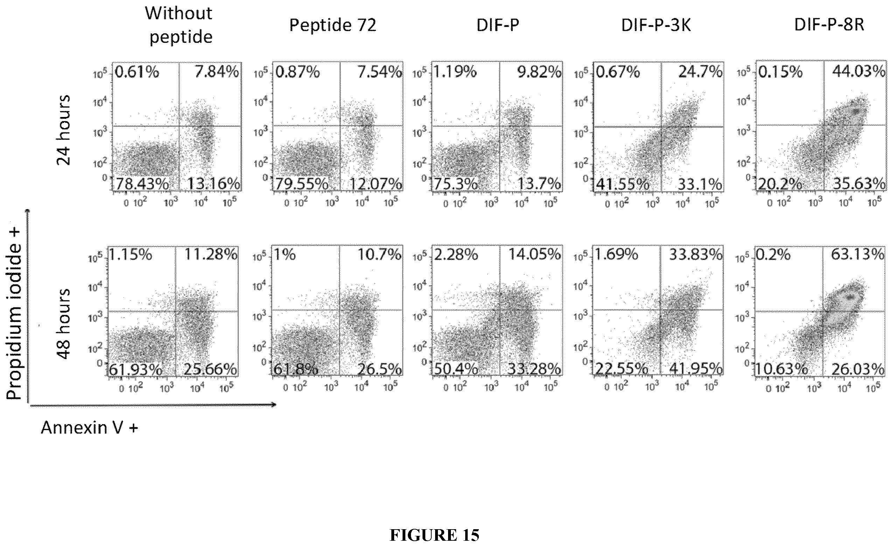

**Figure 15** - Representative dot plots of apoptosis assay of purified neutrophils from NOD bone marrow after culturing them with different peptides. Annexin V and PI positive cells were analysed after culturing 4x10⁵ neutrophils for 24 or 48 hours with peptide 72, or DIF-P, or DIF-P3K, or without stimulus (n = 4).

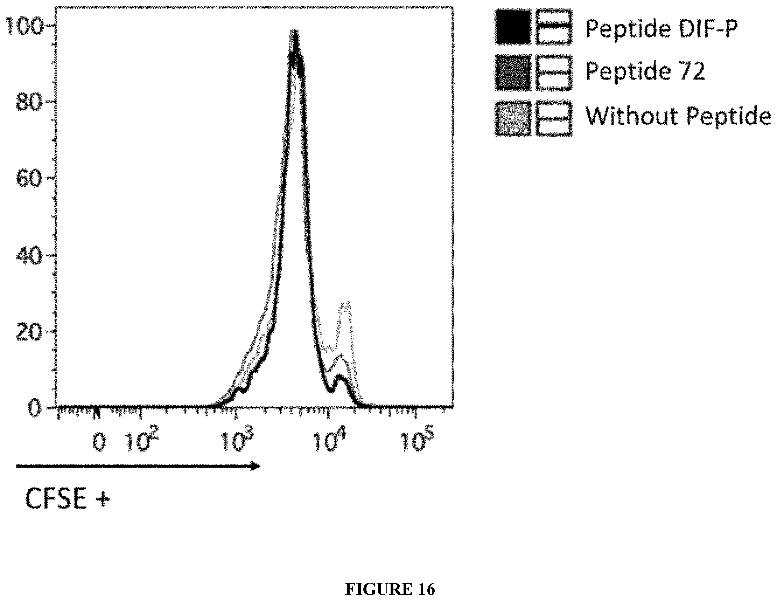

**Figure 16** - Proliferation assay. No proliferation is observed for NOD.RAG-2 -/- mouse splenocytes cultured with DIF-P. Peptide 72 is used as a negative control.

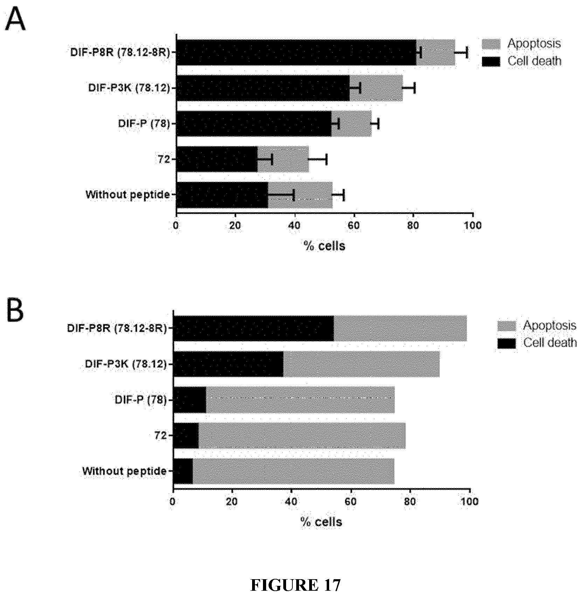

**Figure 17** - Cell death and apoptosis of mouse B16F10 (melanoma) and P815 (mastocitoma) cell lines after culture with different variants of DIF. A) Annexin positive (marker of cellular apoptosis), and Annexin V and propidium iodide double positive (markers of cell death) B16F10 cells, 24 h after culture in presence of different peptide fragments DIF-P, and DIF variants (n=3). B) Annexin positive (marker of cellular apoptosis), and Annexin V and propidium iodide double positive (markers of cell death) P815, cells 24 h after culture in presence of different peptide fragments DIF-P, and DIF variants (n=1).

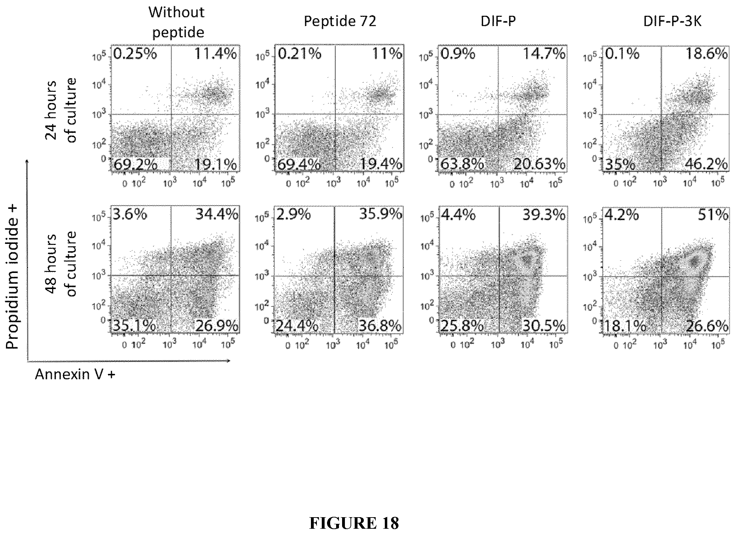

**Figure 18** - Representative dot plots of apoptosis assay of chicken splenocytes after culturing them with different peptides. Annexin V and PI positive cells were analysed after culturing 4x10⁵ cells for 24 hours or 48 hours with peptide 72, or DIF-P, or DIF-P3K, or without stimulus (n = 4).

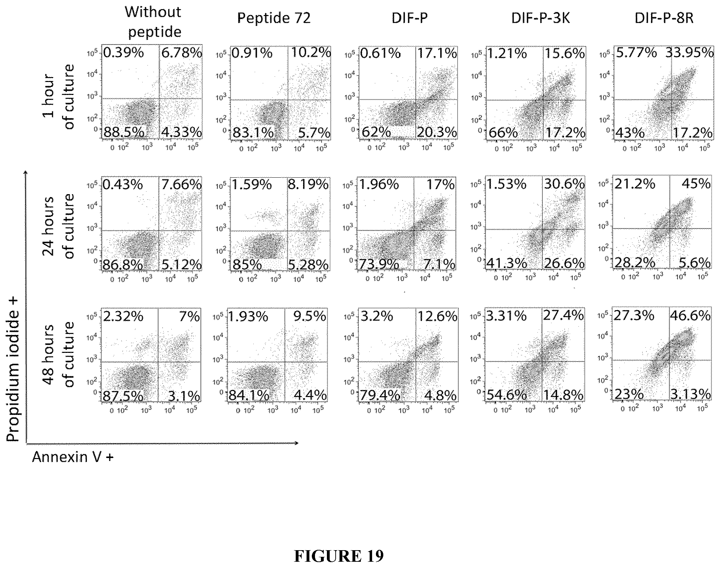

**Figure 19** - Representative dot plots of apoptosis assay of Jurkat cells after culturing them with different peptides. Annexin V and PI positive cells were analysed after culturing 1x10⁵ cells for 1 hour, 24 hours or 48 hours with peptide 72, or DIF-P, or DIF-P3K, or DIF-P8R or without stimulus (n = 4).

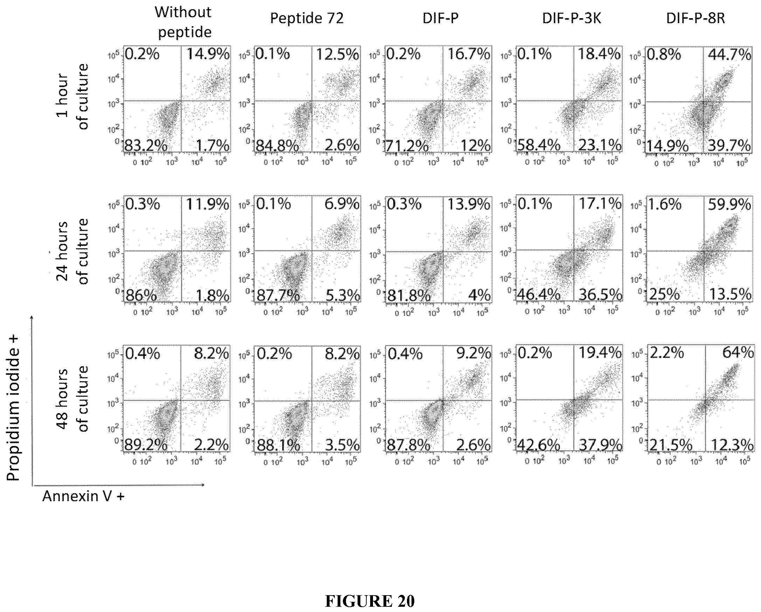

**Figure 20** - Representative dot plots of apoptosis assay of HL-60 cells after culturing them with different peptides. Annexin V and PI positive cells were analysed after culturing 1x10⁵ cells for 1 hour, 24 hours or 48 hours with peptide 72, or DIF-P, or DIF-P3K, or DIF-P8R or without stimulus (n = 4).

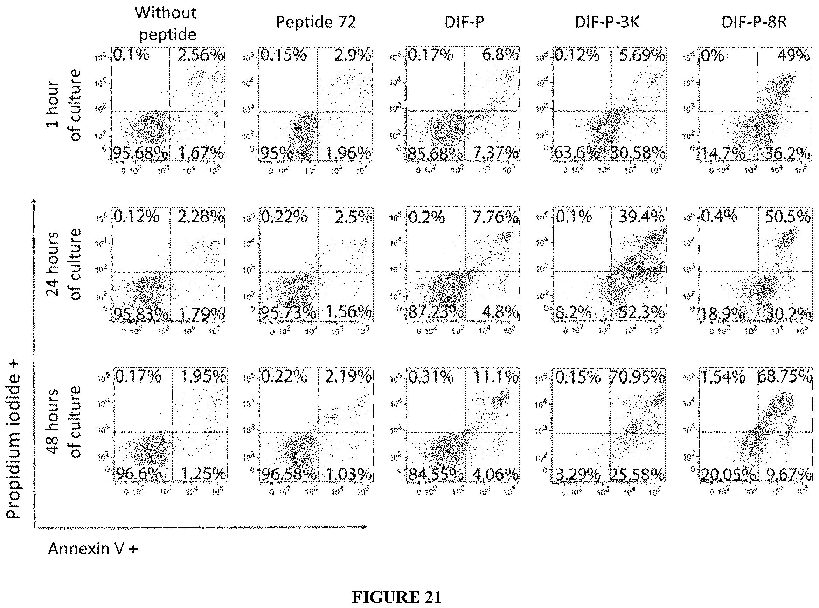

**Figure 21** - Representative dot plots of apoptosis assay of NALM-6 cells after culturing them with different peptides. Annexin V and PI positive cells were analysed after culturing 1x10⁵ cells for 1 hour, 24 hours or 48 hours with peptide 72, or DIF-P, or DIF-P3K, or DIF-P8R or without stimulus (n = 4).

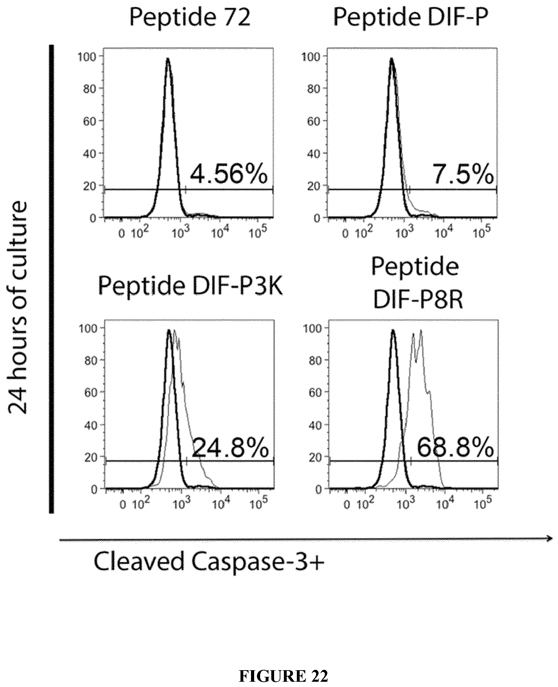

**Figure 22** - Representative images of the expression of cleaved Caspase-3 in A375 cells (human melanoma cell line) cultured with different peptides. A375 cells were cultured with peptide 72, or DIF-P, or DIF-P3K, or DIF-P8R or without stimulus for 24 hours and intracellular staining was performed to analyse the expression of cleaved caspase-3. The line in bold is equivalent to the expression of the control cells without peptide, and clearer curves are equivalent to cells incubated with different peptides (n = 2).

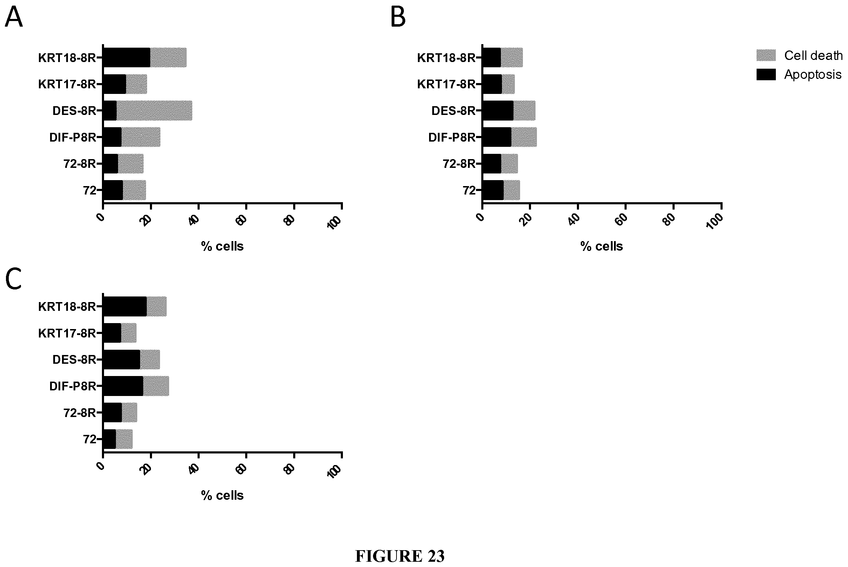

**Figure 23** - Cell death and apoptosis of HEK293, HeLa and RD cells after culture with different variants of DIF (n=1). A) Annexin positive (marker of cellular apoptosis), and Annexin V and propidium iodide double positive (markers of cell death) HEK293 cells, 1 h after culture in presence of different peptide fragments of DIF variants. B) Annexin positive (marker of cellular apoptosis), and Annexin V and propidium iodide double positive (markers of cell death) HeLa cells, 1 h after culture in presence of different peptide fragments of DIF variants. C) Annexin positive (marker of cellular apoptosis), and Annexin V and propidium iodide double positive (markers of cell death) RD cells, 1 h after culture in presence of different peptide fragments of DIF variants.

### DETAILED DESCRIPTION OF THE INVENTION

As explained above, the present invention relates to the surprising finding that peptides derived from known intermediate filaments are capable of inducing apoptosis, pyroptosis and/or necroptosis in all tested types of eukaryotic cells. In addition, in leukocytes, they stimulate pro-inflammatory cytokine secretion, and may thus be useful as new adjuvants in vaccination as well as chemotherapeutic agents for cancer, either alone or in combination with other therapies. The use of the peptides of the invention is particularly indicated for cancers of lymphoid or myeloid origin, or for solid tumours.

### Peptides of the invention

In a first aspect, the invention relates to a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, with the proviso that the peptide sequence is not any of the following:
(i) YQELMNVKLALDIEIATYRR (SEQ ID NO: 2);
(ii) YQDLLNVKMALDIEIATYRR (SEQ ID NO: 3);
(iii) YQDLLNVKLALDIEIATYRR (SEQ ID NO: 4);
(iv) YQDLLNVKMALDVEIATYRR (SEQ ID NO: 5);
(v) YQELMNVKLALDIEIATYRK (SEQ ID NO: 6);
(vi) YQDLLNVKMALDIEIATYRK (SEQ ID NO: 7);
(vii) YQDLLNVKLALDIEIATYRK (SEQ ID NO: 8);
(viii) YQDLLNVKMALDVEIATYRK (SEQ ID NO: 9);
(ix) DYQELMNVKLALDVEIATYR (SEQ ID NO: 10);
(x) VKIALEVEIATY (SEQ ID NO: 11);
(xi) IKSRLEQEIATYRSLLEGQEDHYNNLSASKVL (SEQ ID NO: 12);
(xii) LMDIKSRLEQEIATY (SEQ ID NO: 13).

The term "peptide", as used herein, refers to a sequence of amino acids, analogues or mimetics having substantially similar or identical functionality. The term "peptide" also includes analogues having synthetic and natural amino acids joined together by peptide bonds. A peptide, as used herein and in the claims, is also intended to include analogues, derivatives, salts, retro-inverso isomers, mimics, mimetics, or peptidomimetics thereof. For example, a peptidic structure of a modulator of the invention may be further modified to increase its stability, bioavailability, solubility, etc. "Analog", "derivative" and "mimetic" include molecules which mimic the chemical structure of a peptidic structure and retain the functional properties of the peptidic structure. Approaches to designing peptide analogues, derivatives and mimetics are known in the art. For example, see Farmer, P. S. in Dmg Design (E. J. Ariens, ed.) Academic Press, New York, 1980, vol. 10, pp. 119-143; Ball, J. B. and Alewood, P. F. (1990) J. Mol. Recognition 3:55. Morgan, B. A. and Gainor, J. A. (1989) Ann. Rep. Med. Chem. 24:243; and Freidinger, R. M. (1989) Trends Pharmacol. Sci. 10:270. See also Sawyer, T. K. (1995) Peptidomimetic Design and Chemical Approaches to Peptide Metabolism in Taylor, M. D. and Amidon, G. L. (eds.) Peptide-Based Drug Design: Controlling Transport and Metabolism, Chapter 17; Smith, A. B. 3rd, et al. (1995)J. Am. Chem. Soc. 117:11113-11123; Smith, A. B. 3rd, et al. (1994) J. Am. Chem. Soc. 116:9947-9962; and Hirschman, R., et al. (1993) J. Am. Chem. Soc. 115:12550-12568. A "derivative" (e.g., a peptide or amino acid) includes forms in which one or more reaction groups on the compound have been derivatised with a substituent group.

Examples of peptide derivatives include peptides in which an amino acid side chain, the peptide backbone, or the amino- or carboxy-terminus has been derivatised (e.g., peptidic compounds with methylated amide linkages). An "analogue" of a compound X includes compounds which retain chemical structures necessary for functional activity, yet which also contains certain chemical structures which differ. An example of an analogue of a naturally-occurring peptide is a peptide which includes one or more non-naturally-occurring amino acids. A "mimetic" of a compound includes compounds in which chemical structures of the compound necessary for functional activity have been replaced with other chemical structures which mimic the conformation of the compound. Examples of peptidomimetics include peptidic compounds in which the peptide backbone is substituted with one or more benzodiazepine molecules (see e.g., James, G. L. et al. (1993) Science 260:1937-1942).

The term "amino acid" refers to naturally occurring and synthetic amino acids, as well as amino acid analogues and amino acid mimetics that function in a manner similar to the naturally occurring amino acids. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The term amino acid includes naturally occurring amino acids (Ala, Arg, Asn, Asp, Cys, Gln, Glu, Gly, His, Ile, Leu, Lys, Met, Phe, Pro, Ser, Thr, Trp, Tyr, Val), uncommon natural amino acids, non-natural (synthetic) amino acids. The amino acids are preferably in the L configuration, but also D configuration, or mixtures of amino acids in the D and L configurations. The term "natural amino acids" comprises aliphatic amino acids (glycine, alanine, valine, leucine and isoleucine), hydroxylated amino acids (serine and threonine), sulfured amino acids (cysteine and methionine), dicarboxylic amino acids and their amides (aspartic acid, asparagine, glutamic acid and glutamine), amino acids having two basic groups (lysine, arginine and histidine), aromatic amino acids (phenylalanine, tyrosine and tryptophan) and cyclic amino acids (proline). As used herein the term "non-natural amino acid" refers to a carboxylic acid, or a derivative thereof, substituted at position α with an amine group and being structurally related to a natural aminoacid. Illustrative non-limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allohydroxylysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

Amino acids positions found depicted in brackets indicate that the amino acids found within the brackets can be found as alternatives in the given position. Thus, [(a)/(b)] indicates that the amino acid at said position can be a nonpolar aliphatic amino acid or a polar uncharged amino acid; [K/R] indicates that the amino acid at said position can be Lys or Arg; [(a)/(b)/(c)/(d)] indicates that the amino acid at said position can be a nonpolar aliphatic amino acid, a polar uncharged amino acid, a positively charged amino acid, or an aromatic amino acid; [L] indicates that the amino acid at said position can only be Leu; [(e)] indicates that the amino acid at said position can only be a negatively charged amino acid; [(a)/(b)/(c)] indicates that the amino acid at said position can be a nonpolar aliphatic amino acid, a polar uncharged amino acid, or a positively charged amino acid; [E] indicates that the amino acid at said position can only be Glu; [I] indicates that the amino acid at said position can only be Ile.

As used herein, a nonpolar aliphatic amino acid is selected from the group consisting of Gly, Ala, Val, Pro, Met, Leu, and Ile; a polar uncharged amino acid is selected from the group consisting of Ser, Cys, Thr, Asn, and Gln; a positively charged amino acid is selected from the group consisting of His, Lys, and Arg; an aromatic amino acid is selected from the group consisting of Phe, Tyr, and Trp; and a negatively charged amino acid is selected from the group consisting of Asp, and Glu.

The second region of the peptides of the invention consists of an amino acid sequence of 9 amino acids. The amino acid in position 1 of the second region of the peptides of the invention is a nonpolar aliphatic amino acid or a polar uncharged amino acid; preferably Ala, Ile, Leu, Ser, Thr, or Val, most preferably Val. The amino acid in position 2 of the second region of the peptides of the invention is Lys or Arg; preferably Lys. The amino acid in position 3 of the second region of the peptides of the invention is a nonpolar aliphatic amino acid or a polar uncharged amino acid; preferably Leu, Ile, Met, Ser, Thr, Val, or Ala; most preferably Met or Leu. The amino acid in position 4 of the second region of the peptides of the invention is a nonpolar aliphatic amino acid, a polar uncharged amino acid, a positively charged amino acid, or an aromatic amino acid; preferably Gly, Arg, Ala, His, Lys, Ser, and Phe; most preferably Ala. The amino acid in position 5 of the second region of the peptides of the invention is Leu. The amino acid in position 6 of the second region of the peptides of the invention is a negatively charged amino acid; preferably Asp or Glu, most preferably Asp. The amino acid in position 7 of the second region of the peptides of the invention is a nonpolar aliphatic amino acid, a polar uncharged amino acid, or a positively charged amino acid; preferably Ile, Asn, Val, Met, Lys, Gln, Ala, Leu, Gly or Cys; most preferably Ile or Val. The amino acid in position 8 of the second region of the peptides of the invention is Glu. The amino acid in position 9 of the second region of the peptides of the invention is Ile.

In a particular embodiment, the peptide of the invention has a minimum length of 9 amino acids and a maximum length of 50 amino acids. In another particular embodiment, the peptide of the invention has a minimum length of 12 amino acids and a maximum length of 50 amino acids. In a particular embodiment, the peptide of the invention has a minimum length of 9 amino acids and a maximum length of 20 amino acids. In another particular embodiment, the peptide of the invention has a minimum length of 12 amino acids and a maximum length of 20 amino acids. In another particular embodiment the peptide of the invention has a length of 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids. The first region of the peptide of the invention consists of an amino acid sequence of "n" amino acids, wherein "n" is 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 amino acids. The third region of the peptide of the invention consists of an amino acid sequence of "m" amino acids, wherein "m" is 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 41 amino acids.

In a preferred embodiment, the invention relates to a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14); and
(3) a third region consisting of an amino acid sequence of "m" amino acids, wherein "m" is 0 to 41 amino acids,
wherein said peptide has a minimum length of 9 amino acids and a maximum length of 50 amino acids, with the proviso that the peptide sequence is not any of the following:
(i) YQELMNVKLALDIEIATYRR (SEQ ID NO: 2);
(ii) YQDLLNVKMALDIEIATYRR (SEQ ID NO: 3);
(iii) YQDLLNVKLALDIEIATYRR (SEQ ID NO: 4);
(iv) YQDLLNVKMALDVEIATYRR (SEQ ID NO: 5);
(v) YQELMNVKLALDIEIATYRK (SEQ ID NO: 6);
(vi) YQDLLNVKMALDIEIATYRK (SEQ ID NO: 7);
(vii) YQDLLNVKLALDIEIATYRK (SEQ ID NO: 8);
(viii) YQDLLNVKMALDVEIATYRK (SEQ ID NO: 9);
(ix) DYQELMNVKLALDVEIATYR (SEQ ID NO: 10);
(x) VKIALEVEIATY (SEQ ID NO: 11);
(xi) IKSRLEQEIATYRSLLEGQEDHYNNLSASKVL (SEQ ID NO: 12);
(xii) LMDIKSRLEQEIATY (SEQ ID NO: 13).

In another preferred embodiment, the invention relates to a peptide consisting of, in order going from the N-terminal end to the C-terminal end:
(1) a first region consisting of an amino acid sequence of "n" amino acids, wherein "n" is 0 to 11 amino acids;
(2) a second region consisting of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) a third region consisting of an amino acid sequence of "m" amino acids, wherein "m" is 0 to 11 amino acids,
wherein said peptide has a minimum length of 9 amino acids and a maximum length of 20 amino acids, with the proviso that the peptide sequence is not any of the following:
(i) YQELMNVKLALDIEIATYRR (SEQ ID NO: 2);
(ii) YQDLLNVKMALDIEIATYRR (SEQ ID NO: 3);
(iii) YQDLLNVKLALDIEIATYRR (SEQ ID NO: 4);
(iv) YQDLLNVKMALDVEIATYRR (SEQ ID NO: 5);
(v) YQELMNVKLALDIEIATYRK (SEQ ID NO: 6);
(vi) YQDLLNVKMALDIEIATYRK (SEQ ID NO: 7);
(vii) YQDLLNVKLALDIEIATYRK (SEQ ID NO: 8);
(viii) YQDLLNVKMALDVEIATYRK (SEQ ID NO: 9);
(ix) DYQELMNVKLALDVEIATYR (SEQ ID NO: 10).

In a particular embodiment, the peptide according to the invention is capable of:
(a) inducing apoptosis, pyroptosis and/or necroptosis in leukocytes, and/or
(b) stimulating pro-inflammatory cytokine secretion.
Thus, these peptides have two types of effects depending on their capacity for cell penetration. On the one hand, the peptides are capable of promoting the production of cytokines when they are added to a leukocyte or to a monocyte purified culture, and on the other hand they cause cell death in any population of eukaryotic cells of birds or mammals. The peptides are also capable of inducing cell death in human T-lymphocytes. Due to their endogenous origin, they can be considered "Damage Associated Molecular Pattern" (DAMP).

The term "apoptosis" as used herein relates to a regulated network of biochemical events which lead to a selective form of cell suicide, and is characterised by readily observable morphological and biochemical phenomena, such as the fragmentation of the deoxyribonucleic acid (DNA), condensation of the chromatin, which may or may not be associated with endonuclease activity, chromosome migration, margination in cell nuclei, the formation of apoptotic bodies, mitochondrial swelling, widening of the mitochondrial cristae, opening of the mitochondrial permeability transition pores and/or dissipation of the mitochondrial proton gradient. Methods to determine cell apoptosis are known by the skilled person and include, without limitation, assays that measure DNA fragmentation (including staining of chromosomal DNA after cell permeabilisation), assays that measure the activation of caspases such as caspase 3 (including protease activity assays), assays that measure caspase cleavage products (including detection of PARP and cytokeratin 18 degradation), assays that examine chromatin chromatography (including chromosomal DNA staining), assays that measure DNA strand breaks (nicks) and DNA fragmentation (staggered DNA ends) (including active labelling of cell nick translation or ISNT and active labelling of cells by end labelling or TUNEL), assays that detect phosphatidylserine on the surface of apoptotic cells (including detection of translocated membrane component), assays that measure plasma membrane damage/leakage (including trypan blue exclusion assay and propidium iodide exclusion assay). Exemplary assays include analysis of scatter's parameters of apoptotic cells by flow cytometry, analysis of DNA content by flow cytometry (including DNA staining in a fluorochrome solution such as propidium iodide), fluorochrome labelling of DNA strand breaks by terminal deoxynucleotidyl transferase or TdT-assay, analysis of annexin-V binding by flow cytometry and TUNEL assay.

The term "pyroptosis" as used herein refers to a highly inflammatory form of programmed cell death that occurs most frequently upon infection of immune cells with intracellular pathogens and is characterised by the release of pro-inflammatory cytokines and the swelling and bursting of the infected cell. The released cytokines attract other immune cells to fight the infection and contribute to inflammation in the tissue. Pyroptosis promotes the rapid clearance of various bacterial and viral infections by removing intracellular replication niches and enhancing the host's defensive responses. Pyroptosis requires the function of the enzyme caspase-1, which is activated by a large supramolecular complex termed the pyroptosome (also known as an inflammasome). Unlike apoptosis, cell death by pyroptosis results in plasma-membrane rupture and the release of damage-associated molecular pattern (DAMP) molecules such as ATP, DNA and ASC oligomers (specks) into the extracellular milieu, including cytokines that recruit more immune cells and further perpetuate the inflammatory cascade in the tissue.

The term "necroptosis" as used herein refers to an alternative mode of regulated cell death mimicking features of apoptosis and necrosis. Necroptosis requires protein RIPK3 (previously well recognized as regulator of inflammation, cell survival, and disease) and its substrate MLKL, as the crucial players of this pathway. Necroptosis is induced by toll-like receptor, death receptor, interferon, and some other mediators. The immunogenic nature of necroptosis favours its participation in certain circumstances, such as aiding defence pathogens by the immune system. Necroptosis is well defined as a viral defence mechanism, allowing the cell to undergo "cellular suicide" in a caspase-independent fashion in the presence of viral caspase inhibitors to restrict virus replication. In addition to being a response to disease, necroptosis has also been characterized as a component of inflammatory diseases such as Crohn's disease, pancreatitis, and myocardial infarction.

In the context of the present invention, the term "pro-inflammatory cytokines" refers to cytokines that accelerate inflammation and regulate inflammatory reactions either directly or by their ability to induce the synthesis of cellular adhesion molecules or other cytokines in certain cell types. The major pro-inflammatory cytokines are IL1-α, IL1-β, IL6, and TNF-α.

The peptide of the invention may comprise an additional element or additional elements to deliver the peptide into the cell.

In a particular embodiment, the first region of the peptide of the invention comprises the amino acid sequence L-L-N.

In another particular embodiment, the first region and/or the third region of the peptide of the invention comprise a cell penetrating peptide and/or a signal peptide. Cell-penetrating peptides (CPPs) are short peptides that facilitate cellular intake/uptake of various molecular entities associated with the peptides either through chemical linkage via covalent bonds or through non-covalent interactions. The associated molecular entities range from nanosize particles to small chemical molecules and large fragments of DNA. The function of the CPPs is to deliver the associated entities into cells, a process that commonly occurs through endocytosis. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only apolar residues, with low net charge or have hydrophobic amino acid groups that are crucial for cellular uptake. In a particular embodiment, the cell penetrating peptide is a tumour-penetrating peptide which comprises the 9-amino acid cyclic peptide iRGD (CRGDKGPDC, SEQ ID NO: 16). In another particular embodiment, the cell penetrating peptide comprises a polar amino acid sequence. In a preferred embodiment, the cell penetrating peptide comprises three or more (i.e. 3, 4, 5, 6, 7, 8 or more) sequential Lysine amino acids (K-K-K) or three or more (i.e. 3, 4, 5, 6, 7, 8, or more) sequential Arginine amino acids (R-R-R). In a more preferred embodiment, the cell penetrating peptide comprises eight or more sequential Lysine amino acids (K-K-K-K-K-K-K-K, SEQ ID NO: 17) or eight or more sequential Arginine amino acids (R-R-R-R-R-R-R-R, SEQ ID NO: 18). A signal peptide (sometimes referred to as signal sequence, targeting signal, localisation signal, localisation sequence, transit peptide, leader sequence or leader peptide) is a short peptide (usually 16-30 amino acids long) present at the N-terminus of the majority of newly synthesized proteins that are destined towards the secretory pathway. These proteins include those that reside either inside certain organelles (the endoplasmic reticulum, Golgi or endosomes), secreted from the cell, or inserted into most cellular membranes. In a particular embodiment, the first region and/or the third region of the peptide of the invention comprise a "Nuclear Localization Sequence" (NLS) peptide. In a particular embodiment, the peptide of the invention further comprises a protein tag or is bound to a polypeptide forming a chimeric protein. In a particular example, the peptide of the invention may be bound to an antibody in order to form a chimeric protein.

In a particular embodiment, the peptide of the invention is a peptide of sequence:
(i) LLNVKMALDIEIATYRKLLE (SEQ ID NO: 19);
(ii) LLNVKMALDIEIKKK (SEQ ID NO: 20);
(iii) LLNVKMALDVEIATYRKLLE (SEQ ID NO: 21);
(iv) LLNVKLALDIEIATYRKLLE (SEQ ID NO: 22);
(v) LLNVKMALDIEIAAYRKLLE (SEQ ID NO: 23);
(vi) REYQELLNVKMALDIEIATY (SEQ ID NO: 24);
(vii) LLNVKMALDIEIATYR (SEQ ID NO: 25);
(viii) LLNVKMALDIEIATYKKK (SEQ ID NO: 26);
(ix) LLNVKMALDIEIATKKK (SEQ ID NO: 27);
(x) LLNVKMALDIEIAKKK (SEQ ID NO: 28);
(xi) LNVKMALDIEIATYR (SEQ ID NO: 29);
(xii) NVKMALDIEIATYR (SEQ ID NO: 30);
(xiii) VKMALDIEIATYR (SEQ ID NO: 31);
(xiv) LLNVKMALDVEIATKKK (SEQ ID NO: 32);
(xv) LLNVKMALDVEIKKK (SEQ ID NO: 33);
(xvi) LLNVKMALDVEIAKKK (SEQ ID NO: 34);
(xvii) LLNVKLALDIEIKKK (SEQ ID NO: 35);
(xviii) LLNVKLALDIEIAKKK (SEQ ID NO: 36);
(xix) LLNVKLALDIEIATKKK (SEQ ID NO: 37);
(xx) LLNVKLALDVEIKKK (SEQ ID NO: 38); or
(xxi) LLNVKMALDIEIRRRRRRRR (SEQ ID NO: 39).

All the terms and embodiments described elsewhere herein are equally applicable to these aspects of the invention.

### Nucleic acids, vectors and viruses of the invention

In another aspect, the present invention relates to a nucleic acid encoding for the peptide of the invention, and to an expression cassette, a vector and a virus comprising said nucleic acid.

Preferably, said nucleic acid is a polynucleotide, referring to single-stranded or doublestranded polymers of nucleotide monomers (nucleic acids), including, but not limited to, 2'-deoxyribonucleotides (DNA) and ribonucleotides (RNA) linked by internucleotide phosphodiester bond linkages. In one embodiment, said nucleic acid is codon optimized. In a preferred embodiment, the nucleic acid is codon optimised for expression in humans. Codon-optimized nucleic acids for use according to the present invention can be prepared by replacing the codons of the nucleic acid encoding the immunogen by "humanised" codons (i.e. the codons are those that appear frequently in highly expressed human genes). See Andre S, et al., J. Virol. 1998; 72: 1497-1503. The nucleic acid of this aspect of the invention may require cutting with restriction enzymes in order to it ligate into a vector. This procedure could entail the removal of various terminal nucleotides (e.g. 1, 2, or 3). As such, in one embodiment, the invention relates to said nucleic acid, wherein it has been cut at each end with a restriction enzyme.

In another embodiment, the present invention relates to an expression cassette comprising the nucleic acid of this aspect of the invention, a promoter sequence and a 3'-UTR and optionally a selection marker. Preferably, the promoter sequence is a human cytomegalovirus (CMV) promoter or an early-late p7.5 promotor sequence. Preferably, the 3'-UTR is a bovine growth hormone (BGH) poly-A. The optional selection marker is an antibiotic resistance gene (e.g. kanamycin, ampicilin, tetracycline, spectinomycin) preferably.

In yet another embodiment, the present invention relates to an expression vector comprising the nucleic acid or the expression cassette of this aspect of the invention. The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs which allow for random or site-directed integration of the targeting construct into genomic DNA. Such targeting constructs, preferably, comprise DNA of sufficient length for either homologous recombination or heterologous integration. In a particular embodiment, the vector is an expression vector. The term "expression vector" refers to a replicative DNA construct used for expressing the nucleic acid construct of the invention in a cell, preferably a eukaryotic cell, more preferably a mammalian cell. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

In a particular embodiment, the expression vector is a viral vector or virus comprising the nucleic acid of the invention, or the expression vector of the invention. In a more particular embodiment, the expression vector is a lentiviral vector or an adenoviral vector. The term "lentiviral vector", as used herein, refers to a vector based on a group (or scientific genus) of retroviruses that in nature give rise to slowly developing disease due to their ability to incorporate into a host genome. Modified lentiviral genomes are useful as viral vectors for the delivery of a nucleic acid sequence to a cell. An advantage of lentiviruses for infection of cells is the ability for sustained transgene expression. These viruses include in particular Human Immunodeficiency Virus type 1 (HIV-1), Human Immunodeficiency Virus type 2 (HIV-2), Simian Immunodeficiency Virus (SIV), Feline Immunodeficiency Virus (FIV), Equine Infectious Anaemia Virus (EIAV), Bovine Immunodeficiency Virus (BIV), Visna Virus of sheep (VISNA) and Caprine Arthritis-Encephalitis Virus (CAEV). Recombinant lentiviral vectors are capable of infecting non-dividing cells and can be used for both in vivo and ex vivo gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136, incorporated herein by reference. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific. The lentiviral vectors according to the invention may be genetically modified in such a way that certain genes constituting the native infectious virus are eliminated and replaced with a nucleic acid sequence of interest to be introduced into the target cells. The term "adenoviral vector", as used herein, refers to a vector based on an adenovirus. The term "adenovirus" refers to any virus pertaining to the Adenoviridae family characterized by being a non-enveloped virus with a pseudo-icosahedral nucleocapsid containing a double stranded DNA genome. This term includes any adenovirus capable of infecting a human or an animal, including all groups, subgroups, and serotypes that use CAR, CD46 or desmoglein-2 as receptor for infection of target cells. The term adenovirus includes, without limitation, avian, canine, equine, bovine, ovine, porcine, human or frog adenovirus. In a particular embodiment, the adenovirus is a human adenovirus, i.e. an adenovirus capable of infecting humans. A "serotype" is each of the immunologically different types of adenovirus. There are at least 57 serotypes of human adenovirus that are classified into several subgroups (A to G). In another embodiment, the viral vector is a vector based on a virus or the Parvoviridae family, preferably from the Parvovirinae subfamily, more preferably from the Dependoparvovirus genus, and yet even more preferably an adeno-associated virus.

The expression vector and/or the viral vector should be capable of coding and delivering the 9 amino acid core peptide such that the core peptide may be directly synthesised within the cell, where it would exert its effect.

All the terms and embodiments described elsewhere herein are equally applicable to these aspects of the invention.

### Pharmacological composition of the invention

In a further aspect, the invention refers to a pharmaceutical composition comprising a therapeutically effective amount of the peptide of the invention, the nucleic acid, the expression vector or the virus of the invention, together with at least one pharmaceutically acceptable excipient.

The term "pharmaceutical composition", as used herein, refers to a composition comprising a therapeutically effective amount of the peptide or the nucleic acid construct according to the present invention (or the vector, viral particle or cell comprising said nucleic acid construct) and at least one pharmaceutically acceptable excipient. Pharmaceutical compositions according to the invention can be prepared, for instance, as injectables such as liquid solutions, suspensions, and emulsions. The terms "pharmaceutically acceptable excipient", or "pharmaceutically acceptable carrier," "pharmaceutically acceptable diluent,", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could be selected from the group consisting of sterile liquids, such as water and oils, including those of petroleum, animal, vegetable, or synthetic origin, such as peanut oil, soybean oil, mineral oil, sesame oil, and the like. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

The term "therapeutically effective amount", as used herein, in relation to the compound of the invention, or in relation to the compound, excipient and/or carrier comprised by the pharmaceutical composition of the invention, relates to the sufficient amount of said compound, excipient and/or carrier to provide the desired effect, i.e. to achieve an appreciable prevention, cure, delay, reduction of severity or amelioration of one or more symptoms derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses that may be mentioned in this invention must be considered only as guides for the person skilled in the art, who must adjust the doses depending on the aforementioned variables. In an embodiment, the effective amount produces the amelioration of one or more symptoms of the disease that is being treated. Even though individual needs vary, determination of optimal ranges for therapeutically effective amounts of the compounds according to the invention belongs to the common experience of those experts in the art. In general, the dosage needed to provide an effective treatment, which can be adjusted by one expert in the art, will vary depending on age, health, fitness, sex, diet, weight, degree of alteration of the receptor, frequency of treatment, nature and condition of the injury, nature and extent of impairment or illness, medical condition of the subject, route of administration, pharmacological considerations such as activity, efficacy, pharmacokinetic and toxicology profile of the particular compound used, if using a system drug delivery, and if the compound is administered as part of a combination of drugs. The amount of the compound according to the invention that is therapeutically effective in the prevention and/or treatment of ischemia injury or ischemia/reperfusion injury in a subject can be determined by conventional clinical techniques (see, for example, The Physician's Desk Reference, Medical Economics Company, Inc., Oradell, NJ, 1995, and Drug Facts and Comparisons, Inc., St. Louis, MO, 1993).

All the terms and embodiments described elsewhere herein are equally applicable to these aspects of the invention.

### Medical uses of the peptides of the invention, and of the nucleic acids, vectors, viruses and pharmaceutical compositions of the invention

In a further aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in medicine, with the proviso that the peptide sequence is not VKIALEVEIATY (SEQ ID NO: 11).

In a particular embodiment, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14); and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in medicine, with the proviso that the peptide sequence is not VKIALEVEIATY (SEQ ID NO: 11).

In another particular embodiment, the invention relates to a composition comprising a peptide consisting of, in order going from the N-terminal end to the C-terminal end:
(1) a first region consisting of an amino acid sequence of "n" amino acids, wherein "n" is 0 to 11 amino acids;
(2) a second region consisting of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) a third region consisting of an amino acid sequence of "m" amino acids, wherein "m" is 0 to 11 amino acids,
wherein said peptide has a minimum length of 9 amino acids and a maximum length of 20 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in medicine.

In another further aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use as an adjuvant.

In a particular embodiment, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14); and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use as an adjuvant.

In another particular embodiment, the invention relates to a composition comprising a peptide consisting of, in order going from the N-terminal end to the C-terminal end:
(1) a first region consisting of an amino acid sequence of "n" amino acids, wherein "n" is 0 to 11 amino acids;
(2) a second region consisting of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) a third region consisting of an amino acid sequence of "m" amino acids, wherein "m" is 0 to 11 amino acids,
wherein said peptide has a minimum length of 9 amino acids and a maximum length of 20 amino acids; a nucleic acid encoding said peptide; an expression vector comprising said nucleic acid; or a virus comprising said nucleic acid or said expression vector, or a pharmaceutical composition comprising a therapeutically effective amount of said peptide, said nucleic acid, said expression vector or said virus, together with at least one pharmaceutically acceptable excipient, for use as an adjuvant.

The term "adjuvant", as used herein, refers to a substance which, when added to an immunogenic agent, non-specifically enhances or potentiates an immune response to the agent in a recipient host upon exposure to the mixture. The term "immunogenic agent" or "immunogen", as used herein, refers to an antigen capable of provoking an adaptative immune response if injected by itself. All immunogens are also antigens but not all antigens are immunogens. The term "immunogenic composition", as used herein, refers to a composition that elicits an immune response in a subject that produces antibodies or cell-mediated immune responses against a specific immunogen. Immunogenic compositions can be prepared, for instance, as injectables such as liquid solutions, suspensions, and emulsions. The term "antigenic composition" refers to a composition that can be recognized by a host immune system. For example, an antigenic composition contains epitopes that can be recognized by humoral or cellular components of a host immune system.

In another aspect, the invention relates to a vaccine comprising, as an adjuvant, the peptide of the invention, or the nucleic acid, the expression vector, the virus, or the pharmaceutical composition according to the invention. The term "vaccine", as used herein, refers to a substance or composition that establishes or improves immunity to a particular disease by inducing an adaptive immune response including an immunological memory. A vaccine typically contains an agent that resembles a disease-causing microorganism or a part thereof (e.g. a polypeptide). Vaccines can be prophylactic or therapeutic.

In yet another further aspect, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in the treatment of cancer.

In a particular embodiment, the invention relates to a composition comprising a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14); and
(3) a third region consisting of an amino acid sequence of "m" amino acids, wherein "m" is 0 to 41 amino acids,
wherein said peptide has a minimum length of 9 amino acids and a maximum length of 50 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in the treatment of cancer.

In another particular embodiment, the invention relates to a composition comprising a peptide consisting of, in order going from the N-terminal end to the C-terminal end:
(1) a first region consisting of an amino acid sequence of "n" amino acids, wherein "n" is 0 to 11 amino acids;
(2) a second region consisting of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) a third region consisting of an amino acid sequence of "m" amino acids, wherein "m" is 0 to 11 amino acids,
wherein said peptide has a minimum length of 9 amino acids and a maximum length of 20 amino acids; a nucleic acid encoding said peptide; an expression vector comprising said nucleic acid; a virus comprising said nucleic acid or said expression vector; or a pharmaceutical composition comprising a therapeutically effective amount of said peptide, said nucleic acid, said expression vector or said virus, together with at least one pharmaceutically acceptable excipient, for use in the treatment of cancer.

The term "cancer" or "tumour" as used herein is referred to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. The term cancer includes, without limitation, lung cancer, sarcoma, malignant melanoma, pleural mesothelioma, bladder carcinoma, prostate cancer, pancreas carcinoma, gastric carcinoma, ovarian cancer, hepatoma, breast cancer, colorectal cancer, kidney cancer, oesophageal cancer, suprarenal cancer, parotid gland cancer, head and neck carcinoma, cervix cancer, endometrial cancer, liver cancer, mesothelioma, multiple myeloma, leukaemia, and lymphoma. In a particular embodiment of the invention, the cancer is a lymphoid cancer or a myeloid cancer. In a preferred embodiment, the cancer is multiple myeloma. In another preferred embodiment, the cancer is leukaemia. In yet another preferred embodiment, the cancer is lymphoma. In another particular embodiment, the cancer is a solid tumour.

The term "treatment", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

The compound or pharmaceutical composition for use according to the invention, can be administered to a subject by any suitable route of administration, for example, parenteral (e.g., intramuscular, intravenous, subcutaneous, nasal, etc.), enteral (i.e., oral, rectal, etc.), topical, etc. In a particular embodiment, the compositions for the uses of the invention are administered via an intraperitoneal, intratecal, intravesical, intrapleural, endovenous, intramuscular, subcutaneous, nasal or topical route. In the case of solid tumours, the peptide of the invention can be administered directly into the tumour. In another particular embodiment, the compound or pharmaceutical composition for use according to the invention, is administered parenterally, more preferably by intravenous route. The vectors of the invention, which contain the corresponding polynucleotide of the invention, may be administered directly to a subject by conventional methods. Alternatively, said vectors may be used to transform, or transfect or infect cells, for example, mammal cells, including human, ex vivo, which subsequently will be implanted into a human body or an animal to obtain the desired therapeutic effect. For administration to a human body or an animal, said cells will be formulated in a suitable medium that will have no adverse influence on cell viability.

The terms "subject", "patient" or "individual" are used herein interchangeably to refer to any member of the animal kingdom and can be a vertebrate, such as, a fish, a bird, a reptile, an amphibian or a mammal, including a human, non-human primate, horse, pig, rabbit, dog, sheep, goat, cow, bird, cat, guinea pig or rodent. Preferably, the subject is a mammal, more preferably a human.

All the terms and embodiments described elsewhere herein are equally applicable to these aspects of the invention.

### Uses of the peptides of the invention as programmed cell death-inducing agent

In a final aspect, the invention relates to a use of a peptide according to the present invention as a programmed cell death-inducing agent. Thus, the use of a peptide according to the present invention as an agent capable of inducing programmed cell death by apoptosis, pyroptosis and/or necroptosis is contemplated. In particular, it is envisioned that the peptides of the invention may be useful as reagents for inducing *in vitro* programmed cell death by apoptosis, pyroptosis and/or necroptosis in research and diagnostic applications. A further aspect of the present invention is a method of examining apoptosis including the steps of *in vitro* administering a peptide according to the present invention to a cell culture; inducing apoptosis in the cell culture; and examining progression of apoptosis induced in the cell culture.

All the terms and embodiments described elsewhere herein are equally applicable to these aspects of the invention.

### EXAMPLES

The following invention is hereby described by way of the following examples, which are to be construed as merely illustrative and not limitative of the scope of the invention.

### Materials and methods

### Mice

NOD, NOD-RAG2-/- or C57BL/6 mice were maintained by brother-sister mating under specific pathogen-free conditions at the University of Lleida. This study was carried out in accordance with the principles of the Basel Declaration and recommendations of the Catalan Government (*Generalitat de Catalunya*) concerning the protection of animals for experimentation. The protocol was approved by the Committee on the Ethics of Research in Animal Experimentation of the University of Lleida, Spain. Protocol #: CEEA 02-04/16.

### Human PBMCs and Cell lines

Human Peripheral Blood Mononuclear Cells (PBMCs) from healthy blood donors were supplied by Dr. Jordi Barquinero of the Vall D'Hebron Research Institute (VHIR, Barcelona). NALM-6 (B lymphocyte leukemia) was provided by Dr. Eulàlia Genescà (Institute of Research of Leukemia Josep Carreras, Campus ICO-Germans Trias i Pujol, Badalona), and HL-60 (promyelocytic cell line) and the JURKAT (T lymphocyte) were obtained from the bank of cell lines cells of the IRB Lleida.

### In vitro cultures

HL-60, Jurkat and NALM-6 cells were grown and maintained in complete culture medium in a humidified 5% CO₂ atmosphere at 37°C. Splenocytes from 6-wk-old female NOD, NOD.RAG2-/- and C57B1/6 mice were obtained mechanically disrupting their spleens, and red cells were lysed in 0.87% ammonium chloride solution (Puertas, M. C. et al. Phenotype and Functional Characteristics of Islet-Infiltrating B-Cells Suggest the Existence of Immune Regulatory Mechanisms in Islet Milieu. Diabetes 2007, 56, 940-949). Human splenocytes from buffy coats were collected by density gradient using Histopaque 1077 (Histopaque®-1077, 10771 sigma) as described elsewhere (Johnston, L., Harding, S. A. & La Flamme, A. C. Comparing methods for ex vivo characterization of human monocyte phenotypes and in vitro responses. Immunobiology 2015, 220, 1305-1310). Cells from bone marrow were obtained disrupting femur and tibia as explained elsewhere too (Liu, X. & Quan, N. Immune Cell Isolation from Mouse Femur Bone Marrow. Bio-protocol 5, 2015). Monocytes and neutrophils from bone marrow were purified using the Stemcell kits (19861 and 19762 subsequently) and the kit protocol was followed. Cells were incubated in 96-well tissue culture plates (4x10⁵/well for primary cells and 1x10⁵ for cells lines) at 37°C in 5% CO₂ in complete culture medium using 55 µM of different Peripherin peptides. For proliferation assays, cells were labelled with CFSE (C34554, life technologies). After 1 hour, 24 hours or 48 hours, cells were analysed by flow cytometry and culture supernatants stored at -80°C until use.

### Flow cytometry

For cell death and apoptosis analysis, 400.000 of primary cells or 100.000 of cell line cells were incubated in 96-well plates in 200 µL of complete culture medium for 24 h at 37°C and 5% CO₂ in the absence or presence of 55 µM of different peptides. After 1 hour, 24 hours or 48 hours, apoptosis was measured by CFBlue Annexin V Apoptosis Detection Kit with PI (ANXVKCFB-100T, Immunostep), following kit protocol. For cleaved caspasa-3 intracellular staining, mononuclear cells were permeabilised following the manufacturer's instructions of the FoxP3 Staining Buffer Set (00-5523-00, eBioscience) and the cleaved caspasa-3-Pacific Blue (8788S, Cell Signalling) was used for staining. Cell suspensions were analysed by flow cytometry using FACS CANTO II instrumentation (BD, Biosciences, San Jose, CA) and Flowjo (version 8.7) software.

### Cytokine profile analysis

Culture supernatants from mice proliferation assays were collected and IL-2, IL-4, IL-6, IFN-y, IL-10, TNF-α and IL-17 cytokine amounts analysed by flow cytometry using CBA kit (560485, BD). For human cell cultures, the Legendplex Kit was used (BioLegend, 740502), which studied cytokines IL-12p70, TNF-α, IL-6, IL-4, IL-10, IL-1 β, arginase, IL-1st, IL-12p40, IL-23, IFN-γ and IP-10.

### Intracellular caspase staining with FLICA

Fluorescent labeled inhibitors of caspases (FLICA) probe assays were performed in order to check intracellular activation of specific caspases and confirm previously obtained flow cytometry results by cleaved caspasa-3 antibody. FLICA staining was achieved following manufacturer's instructions, as described elsewhere (Doitsh, G. et al. Cell death by pyroptosis drives CD4 T-cell depletion in HIV-1 infection. Nature 2014, 505, 509-514), after incubating cells with 55 µM of different Peripherin peptides for 1 hour or 24 hours.

### Statistics

PRISM Graphpad software was used for analysis. Statistics were performed using Mann-Whitney U tests. All mean values +- SD is shown.

### Results

### Peptide characterization

A peptide library of 20 amino acid peptides that overlap with the previous and subsequent peptides in 5 amino acids was derived from the fragmentation of the human protein peripherin (Ensembl ID No: ENSG00000135406). Among all the fragments studied, DIF-P (also known as peptide 78, LLNVKMALDIEIATYRKLLE, SEQ ID NO: 19) was identified as a molecule of interest for its ability to induce the production of pro-inflammatory cytokines (Figure 1) and cell death (Figure 2). DIF-P is highly conserved phylogenetically, with 100% sequence homology in mouse, human, rat, camel, beaver, etc. Likewise, it is a highly conserved sequence in different human intermediate filaments:
- Vimentin (100% homology);
- Glial Fibrillary Acidic Protein - GFAP (95% homology);
- Desmin (95% homology);
- Neurofilament (heavy, medium and light) (95% homology);
- Alpha-internexin (95% homology);
- Neurofilament 66 (95% homology).
Peptide 72 is a control peptide (GGYQAGAARLEEELRQLKEE, SEQ ID NO: 75, Peripherin sequence with no stimulating activity)

As shown in table 1 below, results obtained after culturing mouse splenocytes for 24 hours with the different peptide fragments and the analysis of the expression of annexin V as a marker of cell death (apoptosis and/or pyroptosis) suggest that the active part of the peptide fragment is constituted by the following 9 amino acid core sequence: VKMALDIEI (SEQ ID NO: 76).

**Table 1- Peptide core analysis by eliminating one amino acid from each extreme of the sequence. As it can be seeing in this table, DIF-P (peptide 78) needs a 9 amino acid core sequence (highlighted in bold letters) to maintain its apoptotic effect. The apoptotic effect may be lost simply by eliminating any one of these 9 amino acids from the core sequence (Figure 3).**

| **Peptide** | **SEQ ID NO.** | **Sequence** | **Cell Death** |
|---|---|---|---|
| **76** | 40 | MARHLREYQELLN**VKMALDI** | - |
| **77** | 24 | REYQELLN**VKMALDIEI**ATY | + |
| **78** | 19 | LLN**VKMALDIEI**ATYRKLLE | + |
| **78-12** | 20 | LLN**VKMALDIEI**KKK | + |
| **78-13** | 28 | LLN**VKMALDIEI**AKKK | + |
| **78-14** | 27 | LLN**VKMALDIEI**ATKKK | + |
| **78-15** | 26 | LLN**VKMALDIEI**ATYKKK | + |
| **78-16** | 25 | LLN**VKMALDIEI**ATYR | + |
| **78-A** | 29 | LN**VKMALDIEI**ATYRKKK | + |
| **78-B** | 30 | N**VKMALDIEI**ATYR | + |
| **78-C** | 31 | **VKMALDIEI**ATYR | + |
| **78-D** | 41 | **KMALDIEI**ATYR | **-** |
| **79** | 42 | **MALDIEI**ATYRKLLEGEESR | **-** |
| **LMNA** | 77 | LLD**IKLALDMEI**HAYRKLLE | + |
| **KRT84** | 78 | LMN**AKLGLDIEI**ATYRRLLE | + |
| **KRT32** | 79 | LLD**VRARLEGEI**NTYRSLLE | + |
| **KRT13** | 80 | LLD**IKTRLEQEI**ATYRSLLE | + |
| **GFAP** | 81 | LLN**VKLALDIEI**ATYRKLLE | + |
| **NF** | 82 | LLN**VKMALDIEI**AAYRKLLE | + |
| **DES** | 83 | LLN**VKMALDVEI**ATYRKLLE | + |

In view of this result, the inventors investigated whether this 9 amino acid core sequence was also present in other intermediate filaments. Based on the analysis of amino acid variation for the different positions shown in table 2, a consensus sequence can be proposed, as shown in Figure 4.

**Table 2 - Sequences similar to the 9 amino acid core sequence found in other types of intermediate filaments. Other intermediate filaments were analysed in order to find sequences which are similar to the 9 amino acid core sequence found in DIF-P amino acid sequence (DIF sequence).**

| **Peptide Sequence** | **SEQ ID NO:** | **Isoelectric Point** | **Molecular weight** | **Intermediate filaments** |
|---|---|---|---|---|
| **AKLGLDIEI** | 43 | 4.37 | 971.16 | KRT84 (IF-II) |
| **IKLALDVEI** | 44 | 4.37 | 1013.24 | KRT75 (IF-II) |
| **IKLALDMEI** | 45 | 4.37 | 1045.30 | LaminA LaminC1, LaminC2 (IF-V) |
| **LKLALDMEI** | 46 | 4.37 | 1045.30 | KRT71, KRT72, KRT74 (IF-II) |
| **SKLGLDIEI** | 47 | 4.37 | 987.16 | KRT81, KRT82, KRT83, KRT85, KRT86 (IF-II) |
| **TKLALDLEI** | 48 | 4.37 | 1015.21 | KRT1 (IF-II) |
| **TKLALDVEI** | 49 | 4.37 | 1001.19 | KRT5 (IF-II) |
| **TKLSLDVEI** | 50 | 4.37 | 1017.19 | KRT77, KRT78 (IF-II) |
| **VKLALDIEI** | 51 | 4.37 | 1013.24 | GFAP (IF-III), KRT8 (IF-II), KRT4 (IF-II), KRT7 (IF-II), KRT80 (IF-II) |
| **VKLALDMEI** | 52 | 4.37 | 1031.28 | LaminB1, LaminB2 (IF-V) |
| **VKLALDVEI** | 53 | 4.37 | 999.22 | KRT79, KRT3, KRT76, KRT2, KRT6A, KRT6B, KRT6C (IF-II) |
| **VKLSLDIEI** | 54 | 4.37 | 1029.24 | KRT73 (IF-II) |
| **VKMALDIEI** | 55 | 4.37 | 1031.28 | Peripherin y Vimentin (IF-III); a-internexin, NF-H, NF-M y NF-L (IF-IV) |
| **VKMALDVEI** | 56 | 4.37 | 1017.25 | Desmin (IF-III) |
| **VKSRLECEI** | 57 | 6.11 | 1076.28 | KRT39 (IF-I) |
| **VKTRLENEI** | 58 | 6.11 | 1101.27 | KRT41 (IF-I) |
| **VKTRLEQEI** | 59 | 6.11 | 1115.30 | KRT14, KRT15, KRT16, KRT17 (IF-I) |
| **VRARLECEI** | 60 | 6.11 | 1088.29 | KRT31, KRT33A, KRT33B, KRT34, KRT35 (IF-I) |
| **VRARLEGEI** | 61 | 6.11 | 1042.20 | KRT32 (IF-I) |
| **VKARLEGEI** | 62 | 6.11 | 1014.19 | KRT36 (IF-I), KRT80 (IF-II) |
| **VKARLELEI** | 63 | 6.11 | 1070.30 | KRT12 (IF-I) |
| **VKARLENEI** | 64 | 6.11 | 1071.24 | KRT37 (IF-I) |
| **VKIFLEKEI** | 65 | 6.11 | 1118.38 | KRT26 (IF-I) |
| **IKTRLEQEI** | 66 | 6.14 | 1129.32 | KRT13, KRT20 (IF-I) |
| **IKIRLENEI** | 67 | 6.14 | 1127.35 | KRT10 (IF-I) |
| **IKTRLEVEI** | 68 | 6.14 | 1100.32 | KRT24 (IF-I) |
| **IKVKLEAEI** | 69 | 6.14 | 1042.28 | KRT18 (IF-I) |
| **IKSRLEQEI** | 70 | 6.14 | 1115.29 | KRT19 (IF-I) |
| **VKVHLEKEI** | 71 | 6.73 | 1094.32 | KRT27, KRT28 (IF-I) |
| **IKLHLEKEI** | 72 | 6.76 | 1122.37 | KRT25 (IF-I) |
| **IKTHLEKEI** | 73 | 6.76 | 1110.32 | KRT23 (IF-I) |
| **IKMRLEKEI** | 74 | 8.59 | 1159.45 | KRT9 (IF-I) |

However, further experiments demonstrated that the 9 amino acid core sequence is not active by itself when it is directly added to a cell culture. Detailed analyses carried out with the Peripherin-derived peptide and with those peptides corresponding to Desmin and Glial Fibrillary Acidic Protein [Per9: VKMALDIEI (SEQ ID NO: 55); Des9: VKMALDVEI (SEQ ID NO: 56); FAP9: VKLALDIEI (SEQ ID NO: 51)] turned out not to be positive (i.e. none of the 9 amino acid peptides was able to induce the expression of annexin V in different populations of leukocytes), indicating that the presence of additional amino acids at the N-terminal and/or C-terminal ends, regardless of their amino acid sequence, are necessary for their correct functionality (in order to fix peptide in the cytoplasm membrane and induce their internalization into the cell citoplasm).

Specifically, the addition of a minimum of 3 amino acids at each end seems to be the most suitable functional form, particularly when the C-terminal end has a cell penetrating peptide with an amino acid composition that contains a high relative abundance of positively charged amino acids such as lysine or arginine. For example, the inventors observed that the peptide sequences DIF-P3K, (also known as peptide 78-12-3K) LLNVKMALDIEIKKK (SEQ ID NO: 20) and DIF-P8R, (also known as 78-12-8R) LLNVKMALDIEIRRRRRRRR (SEQ ID NO: 39) can cause cell death (determined by cleaved Caspase-3 expression) at 22% and 55% after only 1 hour, or at 40% and 85% after 24 hours in a cell culture of NALM-6 cells, a model for human acute lymphoblastic leukaemia (Figure 5).

The cytolytic activity (induction of apoptosis and/or pyroptosis) and production of pro-inflammatory cytokines in cell cultures have been analysed for peptides DIF-P, DIF-P3K and DIF-P8R, using peptide 72 as a negative control, in cultures of human PBMC (peripheral blood mononuclear cells). The induction of apoptosis and/or pyroptosis, and cell death was detected by flow cytometry analysis, determining the expression (presence) of annexin V in the cell membrane, and the staining of the cell nucleus with propidium iodide, respectively (Figure 6). The presence of cellular pyroptosis is confirmed by the analysis of Caspase-1 expression after incubation with the stimulus for an hour (Figure 7), whereas apoptotic cell death is revealed by the analysis of Caspase-3 expression after incubation with the stimulus for an hour (Figure 8). The production of pro-inflammatory cytokines (IL-6, IL-1β, TNF-α, IL-12p40, IL-23, IP-10, IFN-y) is determined after incubation with the stimulus after 24 or 48 hours (Figure 9).

The results obtained indicate that peptides DIF-P and DIF-P3K and DIF-P8R induce apoptosis and/or pyroptosis, while they also induce secretion of pro-inflammatory cytokines. However, the degree of induction of cell death by apoptosis and/or pyroptosis, and induction of cytokine secretion varies from one peptide to another. Thus, peptide DIF-P induces less cell death by apoptosis compared to peptides DIF-P3K or DIF-P8R, whereas peptide DIF-P3K induces a significant percentage of pyroptosis compared to peptide DIF-P and almost no pyroptosis is induced by peptide DIF-P8R. In contrast, a significant production of pro-inflammatory cytokines by leukocytes that remain alive at the end of the culture is observed in cultures exposed to peptide DIF-P, while peptide DIF-P3K is the least efficient in inducing cytokine secretion. In general, peptides DIF-P3K and DIF-P8R induce rapid cell death, even in malignant cells, but a lower production of pro-inflammatory cytokines than peptide DIF-P.

### Induction of cell death and stimulation of the secretion of pro-inflammatory cytokines in mouse splenocytes or bone marrow cells

NOD.RAG-2 -/- mice are carriers of a mutation in homozygosis in the Rag-2 gene (Recombination Activation Gene-2) that makes it impossible to rearrange the V(D)J regions of the immunoglobulin genes and the BCR/TCRs. As a consequence in these mice there is no maturation of B and T lymphocytes, and therefore, a total absence of these cells from acquired immunity. This means that in the secondary lymphoid organs of these animals, as in the spleen, the proportion of other cellular subpopulations of the immune system, such as macrophages and DCs, is different to that observed under normal conditions (i.e. wild-type mouse). This makes it possible to study these populations more efficiently.

According to the data obtained in cultures at 24h and 48h of NOD.RAG-2 -/- mouse splenocytes with peptides DIF-P, DIF-P3K and DIF-P8R it can be seen that release of pro-inflammatory cytokines such as IL-6 and IL-4 to the culture medium is particularly significant for peptide DIF-P8R, whereas peptides DIF-P and DIF-P3K are more efficient at inducing release of TNF-α (Figure 10). However, in cultures at 24h and 48h of NOD.RAG-2 -/- mouse bone marrow cells with peptides DIF-P, DIF-P3K and DIF-P8R, both peptides DIF-P3K and DIF-P8R appear to be similarly efficient at stimulating the release of inflammatory cytokines IL-6 and IL-4 to the culture medium (Figure 11). Interestingly, when monocytes and neutrophils are purified from the bone marrow of NOD mice, the stimulation of inflammatory cytokines IL-6 and IL-4 to the culture medium is remarkably high for peptide DIF-P3K for monocytes and still significant for neutrophils, when compared to the other two peptides (Figure 12). In terms of cell death, both peptides DIF-P3K and DIF-P8R are more efficient than peptide DIF-P at inducing apoptosis and/or pyroptosis in bone marrow cells (Figure 13), purified neutrophils (Figure 14) and monocytes (Figure 15).

The inventors also studied the ability of peptide DIF-P to induce the proliferation of B lymphocytes, macrophages, and DCs. The results indicated that there were no differences in proliferation between the cells cultured with peptide DIF-P (LLNVKMALDIEIATYRKLLE, SEQ ID NO: 19, i.e., sequence of interest) and the control peptides (GGYQAGAARLEEELRQLKEE, SEQ ID NO: 75, i.e. Peptide 72, Peripherin sequence with no stimulating activity) in *in vitro* cultures and, therefore, it follows that peptide DIF-P does not stimulate leukocyte proliferation (Figure 16).

### Induction of cell death in other lineages and in different species

Induction of cell death by the peptides of the invention was also demonstrated in cell cultures of different mouse lineages, or in cell cultures corresponding to different species (i.e., chicken). Mouse B16F10 (melanoma) and P815 (mastocitoma) cell lines were cultured for 24 h with DIF-P (LLNVKMALDIEIATYRKLLE, SEQ ID NO: 19); DIF-P3K (LLNVKMALDIEIKKK, SEQ ID NO: 20); DIF-P8R (LLNVKMALDIEIRRRRRRRR; SEQ ID NO: 39); and Peptide 72 (GGYQAGAARLEEELRQLKEE; SEQ ID NO: 75) or no stimulus as a control (Figure 17). Chicken splenocytes were cultured for 24 hours or 48 hours with DIF-P (LLNVKMALDIEIATYRKLLE, SEQ ID NO: 19); DIF-P3K (LLNVKMALDIEIKKK, SEQ ID NO: 20); and Peptide 72 (GGYQAGAARLEEELRQLKEE; SEQ ID NO: 75), or without stimulus (Figure 18). These results indicate that the peptides of the invention are generally capable of inducing cell death in wide range of metazoan cells (i.e., vertebrate animal cells).

### Induction of cell death in human cell lines

Cell death studies are complemented with the analysis of the capacity of peptides DIF-P, DIF-P3K and DIF-P8R to induce apoptosis, pyroptosis and/or necroptosis in cells from different cell lines of human leukaemia. The cell lines are:
i. Jurkat T-cells: A T-cell leukaemia model;
ii. HL-60 cells: An acute myeloid leukaemia (AML) model;
iii. NALM-6 cells: An acute lymphoblastic leukaemia model (ALL).

Figure 19 shows the mortality percentage in Jurkat-T cells after the addition in the culture of peptides DIF-P, DIF-P3K and DIF-P8R, at times of 1, 24 and 48 hours, by flow cytometry. In Jurkat-T cells, peptide DIF-P induces mortality of 38% (1h), 26% (24h) and 20% (48h) of the cells, while peptide DIF-P3K induces a greater percentage of cell death: 44% (1h), 58% (24h) and 45% (48h) and peptide DIF-P8R induces the greatest percentage of cell death: 57% (1h), 71% (24h) and 77% (48h).

Figure 20 shows the mortality percentage in HL-60 cells after the addition in the culture of peptides DIF-P, DIF-P3K and DIF-P8R, at times of 1, 24 and 48 hours, by flow cytometry. In HL-60 cells, peptide DIF-P induces mortality of 28% (1h), although the total number of dead cells is lower at 24h (18%) and at 48h (12%), probably due to cell proliferation. Peptide DIF-P3K induces a greater percentage of cell death: 41% (1h), 53% (24h) and 57% (48h) and peptide DIF-P8R induces the greatest percentage of cell death: 85% (1h), 75% (24h) and 78% (48h).

Figure 21 shows the mortality percentage in NALM-6 cells after the addition in the culture of peptides DIF-P, DIF-P3K and DIF-P8R, at times of 1, 24 and 48 hours, by flow cytometry. In NALM-6 cells peptide DIF-P induces mortality of 14% (1h), 12% (24h) and 15% (48h) of the cells, while peptide DIF-P3K induces 36% (1h), 91% (24h) and 96% (48h) and peptide DIF-P8R induces 85% (1h), 81% (24h) and 80% (48h).

The capacity of the peptides to induce cell death is also demonstrated on a cell line of human melanoma (A375 cells) and on cell lines such as HEK293, HeLa and RD cells.

Figure 22 shows the expression of cleaved Caspase-3 in A375 cells after a 24-hour culture with the following peptides: DIF-P (LLNVKMALDIEIATYRKLLE, SEQ ID NO: 19); DIF-P3K (LLNVKMALDIEIKKK, SEQ ID NO: 20); DIF-P8R (LLNVKMALDIEIRRRRRRRR; SEQ ID NO: 39); and Peptide 72 (GGYQAGAARLEEELRQLKEE; SEQ ID NO: 75).

Figure 23 shows cell death and apoptosis for cell lines HEK293, HeLa and RD cells after a 1-hour culture with the following peptides: KRT18-8R (LLNIKVKLEAEIRRRRRRRR, SEQ ID NO: 84), KRT17-8R (LLDVKTRLEQEIRRRRRRRR, SEQ ID NO: 85), DES-8R (LLNVKMALDVEIRRRRRRRR, SEQ ID NO: 86), DIF-P8R (LLNVKMALDIEIRRRRRRRR; SEQ ID NO: 39); Peptide 72-8R (GGYQAGAARLEEELRQLKEERRRRRRRR; SEQ ID NO: 76); and Peptide 72 (GGYQAGAARLEEELRQLKEE; SEQ ID NO: 75).

## Claims

1. A peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids,
with the proviso that the peptide sequence is not any of the following:
(xiii) YQELMNVKLALDIEIATYRR (SEQ ID NO: 2);
(xiv) YQDLLNVKMALDIEIATYRR (SEQ ID NO: 3);
(xv) YQDLLNVKLALDIEIATYRR (SEQ ID NO: 4);
(xvi) YQDLLNVKMALDVEIATYRR (SEQ ID NO: 5);
(xvii) YQELMNVKLALDIEIATYRK (SEQ ID NO: 6);
(xviii) YQDLLNVKMALDIEIATYRK (SEQ ID NO: 7);
(xix) YQDLLNVKLALDIEIATYRK (SEQ ID NO: 8);
(xx) YQDLLNVKMALDVEIATYRK (SEQ ID NO: 9);
(xxi) DYQELMNVKLALDVEIATYR (SEQ ID NO: 10);
(xxii) VKIALEVEIATY (SEQ ID NO: 11);
(xxiii) IKSRLEQEIATYRSLLEGQEDHYNNLSASKVL (SEQ ID NO: 12);
(xxiv) LMDIKSRLEQEIATY (SEQ ID NO: 13).

2. The peptide according to claim 1, wherein the sequence of 9 amino acids in the second region is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14).

3. The peptide according to claim 2, wherein:
(1) the first region consists of an amino acid sequence of "n" amino acids, wherein "n" is 0 to 8 amino acids;
(2) the second region consists of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) the third region consists of an amino acid sequence of "m" amino acids, wherein "m" is 0 to 8 amino acids,
wherein said peptide has a minimum length of 12 amino acids and a maximum length of 20 amino acids.

4. The peptide according to any one of claims 1 to 3 wherein the first region and/or the third region comprise a cell penetrating peptide and/or a signal peptide; preferably wherein
- the cell penetrating peptide and/or the signal peptide comprise iRGD (CRGDKGPDC, SEQ ID NO: 16) and/or wherein
- the cell penetrating peptide and/or the signal peptide comprise a polar amino acid sequence, more preferably three or more sequential Lysine amino acids (K-K-K) or three or more sequential Arginine amino acids (R-R-R); and most preferably eight or more sequential Lysine amino acids (K-K-K-K-K-K-K-K, SEQ ID NO: 17) or eight or more sequential Arginine amino acids (R-R-R-R-R-R-R-R, SEQ ID NO: 18).

5. A composition comprising:
a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids,
for use in medicine,
with the proviso that the peptide sequence is not VKIALEVEIATY (SEQ ID NO: 11).

6. The composition for use according to claim 5, wherein the sequence of 9 amino acids in the second region of the peptide is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14).

7. The composition for use according to claim 6, wherein:
(1) the first region in the peptide consists of an amino acid sequence of "n" amino acids, wherein "n" is 0 to 11 amino acids;
(2) the second region in the peptide consists of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) the third region in the peptide consists of an amino acid sequence of "m" amino acids, wherein "m" is 0 to 11 amino acids,
wherein said peptide has a minimum length of 9 amino acids and a maximum length of 20 amino acids.

8. A composition comprising:
a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids,
for use as an adjuvant.

9. The composition for use according to claim 8, wherein the sequence of 9 amino acids in the second region of the peptide is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14).

10. The composition for use according to claim 9, wherein:
(1) the first region in the peptide consists of an amino acid sequence of "n" amino acids, wherein "n" is 0 to 11 amino acids;
(2) the second region in the peptide consists of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) the third region in the peptide consists of an amino acid sequence of "m" amino acids, wherein "m" is 0 to 11 amino acids,
wherein said peptide has a minimum length of 9 amino acids and a maximum length of 20 amino acids.

11. A composition comprising:
a peptide consisting of, in order going from the N-terminal end to the C-terminal end:
(1) a first region consisting of an amino acid sequence of "n" amino acids, wherein "n" is 0 to 41 amino acids;
(2) a second region consisting of an amino acid sequence of 9 amino acids; wherein the sequence of 9 amino acids is [(a)/(b)]-[K/R]-[(a)/(b)]-[(a)/(b)/(c)/(d)]-[L]-[(e)]-[(a)/(b)/(c)]-[E]-[I] (SEQ ID NO: 1), wherein (a) is a nonpolar aliphatic amino acid, (b) is a polar uncharged amino acid, (c) is a positively charged amino acid, (d) is an aromatic amino acid, (e) is a negatively charged amino acid; and
(3) a third region consisting of an amino acid sequence of "m" amino acids, wherein "m" is 0 to 41 amino acids,
wherein said peptide has a minimum length of 9 amino acids and a maximum length of 50 amino acids, for use in the treatment of cancer.

12. The composition for use according to claim 11, wherein the sequence of 9 amino acids in the second region of the peptide is [A/I/L/S/T/V]-[K/R]-[L/I/M/S/T/V/A]-[G/R/A/H/K/S/F]-[L]-[D/E]-[I/N/V/M/K/Q/A/L/G/C]-[E]-[I] (SEQ ID NO: 14).

13. The composition for use according to claim 12, wherein:
(1) the first region of the peptide consists of an amino acid sequence of "n" amino acids, wherein "n" is 0 to 11 amino acids;
(2) the second region of the peptide consists of an amino acid sequence of 9 amino acids; wherein the sequence of 9 amino acids is [V]-[K]-[M/L]-[A]-[L]-[D]-[I/V]-[E]-[I] (SEQ ID NO: 15); and
(3) the third region of the peptide consists of an amino acid sequence of "m" amino acids, wherein "m" is 0 to 11 amino acids,
wherein said peptide has a minimum length of 9 amino acids and a maximum length of 20 amino acids.

14. The composition for use according to any one of claims 11 to 13, wherein the cancer is a lymphoid cancer or a myeloid cancer, or wherein the cancer is a solid tumour.

15. Use of a peptide according to any one of claims 1 to 4 as a programmed cell death-inducing agent.


SEQUENCE LISTING

<110>  UNIVERSITAT DE LLEIDA

<120>  INTERMEDIATE FILAMENT-DERIVED PEPTIDES AND THEIR USES

<130>  P16561EP00

<160>  86

<170>  PatentIn version 3.5

<210>  1
<211>  9
<212>  PRT
<213>  Artificial Sequence

<220>
<223>  Core peptide


<220>
<221>  VARIANT
<222>  (1)..(1)
<223>  /replace= "Gly" /replace= "Ala" /replace= "Pro" /replace= "Met"
       /replace= "Leu" /replace= "Ile" /replace= "Ser" /replace= "Cys"
       /replace= "Thr" /replace= "Asn" /replace= "Gln"

<220>
<221>  VARIANT
<222>  (2)..(2)
<223>  /replace= "Arg"

<220>
<221>  VARIANT
<222>  (3)..(3)
<223>  /replace= "Gly" /replace= "Ala" /replace= "Val" /replace= "Pro"
       /replace= "Leu" /replace= "Ile" /replace= "Ser" /replace= "Cys"
       /replace= "Thr" /replace= "Asn" /replace= "Gln"

<220>
<221>  VARIANT
<222>  (4)..(4)
<223>  /replace="Gly" /replace="Val" /replace="Pro" /replace="Met"
       /replace="Leu" /replace="Ile" /replace="Ser" /replace="Cys"
       /replace="Thr" /replace="Asn" /replace="Gln" /replace="His"
       /replace="Lys" /replace="Arg" /replace="Phe" /replace="Tyr"


<220>
<221>  VARIANT
<222>  (6)..(6)
<223>  /replace= "Glu"

<220>
<221>  VARIANT
<222>  (7)..(7)
<223>  /replace= "Gly" /replace= "Ala" /replace= "Val" /replace= "Pro"
       /replace= "Met" /replace= "Leu" /replace= "Ser" /replace= "Cys"
       /replace= "Thr" /replace= "Asn" /replace= "Gln" /replace= "His"
       /replace= "Lys" /replace= "Arg"

<400>  1

Val Lys Met Ala Leu Asp Ile Glu Ile
1               5

<210>  2
<211>  20
<212>  PRT
<213>  Artificial Sequence

<220>
<223>  Excluded peptide (proviso)

<400>  2

Tyr Gln Glu Leu Met Asn Val Lys Leu Ala Leu Asp Ile Glu Ile Ala
1               5                   10                  15

Thr Tyr Arg Arg
            20

<210>  3
<211>  20
<212>  PRT
<213>  Artificial Sequence

<220>
<223>  Excluded sequence (proviso)

<400>  3

Tyr Gln Asp Leu Leu Asn Val Lys Met Ala Leu Asp Ile Glu Ile Ala
1               5                   10                  15

Thr Tyr Arg Arg
            20

<210>  4
<211>  20
<212>  PRT
<213>  Artificial Sequence

<220>
<223>  Excluded sequence (proviso)

<400>  4

Tyr Gln Asp Leu Leu Asn Val Lys Leu Ala Leu Asp Ile Glu Ile Ala
1               5                   10                  15

Thr Tyr Arg Arg
            20

<210>  5
<211>  20
<212>  PRT
<213>  Artificial Sequence

<220>
<223>  Excluded sequence (proviso)

<400>  5

Tyr Gln Asp Leu Leu Asn Val Lys Met Ala Leu Asp Val Glu Ile Ala
1               5                   10                  15

Thr Tyr Arg Arg
            20

<210>  6
<211>  20
<212>  PRT
<213>  Artificial Sequence

<220>
<223>  Excluded sequence (proviso)

<400>  6

Tyr Gln Glu Leu Met Asn Val Lys Leu Ala Leu Asp Ile Glu Ile Ala
1               5                   10                  15

Thr Tyr Arg Lys
            20

<210>  7
<211>  20
<212>  PRT
<213>  Artificial Sequence

<220>
<223>  Excluded sequence (proviso)

<400>  7

Tyr Gln Asp Leu Leu Asn Val Lys Met Ala Leu Asp Ile Glu Ile Ala
1               5                   10                  15

Thr Tyr Arg Lys
            20

<210>  8
<211>  20
<212>  PRT
<213>  Artificial Sequence

<220>
<223>  Excluded sequence (proviso)

<400>  8

Tyr Gln Asp Leu Leu Asn Val Lys Leu Ala Leu Asp Ile Glu Ile Ala
1               5                   10                  15

Thr Tyr Arg Lys
            20

<210>  9
<211>  20
<212>  PRT
<213>  Artificial Sequence

<220>
<223>  Excluded sequence (proviso)

<400>  9

Tyr Gln Asp Leu Leu Asn Val Lys Met Ala Leu Asp Val Glu Ile Ala
1               5                   10                  15

Thr Tyr Arg Lys
            20

<210>  10
<211>  20
<212>  PRT
<213>  Artificial Sequence

<220>
<223>  Excluded sequence (proviso)

<400>  10

Asp Tyr Gln Glu Leu Met Asn Val Lys Leu Ala Leu Asp Val Glu Ile
1               5                   10                  15

Ala Thr Tyr Arg
            20

<210>  11
<211>  12
<212>  PRT
<213>  Artificial Sequence

<220>
<223>  Excluded sequence (proviso)

<400>  11

Val Lys Ile Ala Leu Glu Val Glu Ile Ala Thr Tyr
1               5                   10

<210>  12
<211>  32
<212>  PRT
<213>  Artificial Sequence

<220>
<223>  Excluded sequence (proviso)

<400>  12

Ile Lys Ser Arg Leu Glu Gln Glu Ile Ala Thr Tyr Arg Ser Leu Leu
1               5                   10                  15

Glu Gly Gln Glu Asp His Tyr Asn Asn Leu Ser Ala Ser Lys Val Leu
            20                  25                  30

<210>  13
<211>  15
<212>  PRT
<213>  Artificial Sequence

<220>
<223>  Excluded sequence (proviso)

<400>  13

Leu Met Asp Ile Lys Ser Arg Leu Glu Gln Glu Ile Ala Thr Tyr
1               5                   10                  15

<210>  14
<211>  9
<212>  PRT
<213>  Artificial Sequence

<220>
<223>  Core peptide

<220>
<221>  VARIANT
<222>  (1)..(1)
<223>  /replace= "Ala" /replace= "Ile" /replace= "Leu" /replace= "Ser"
       /replace= "Thr"

<220>
<221>  VARIANT
<222>  (2)..(2)
<223>  /replace= "Arg"

<220>
<221>  VARIANT
<222>  (3)..(3)
<223>  /replace= "Leu" /replace= "Ile" /replace= "Ser" /replace= "Thr"
       /replace= "Val" /replace= "Ala"

<220>
<221>  VARIANT
<222>  (4)..(4)
<223>  /replace= "Gly" /replace= "Arg" /replace= "His" /replace= "Lys"
       /replace= "Ser" /replace= "Phe"

<220>
<221>  VARIANT
<222>  (6)..(6)
<223>  /replace= "Glu"

<220>
<221>  VARIANT

<222>  (7)..(7)
<223>  /replace= "Asn" /replace= "Val" /replace= "Met" /replace= "Lys"
       /replace= "Gln" /replace= "Ala" /replace= "Leu" /replace= "Gly"
       /replace= "Cys"

<400>  14

Val Lys Met Ala Leu Asp Ile Glu Ile
1               5


<210>  15
<211>  9
<212>  PRT
<213>  Artificial Sequence

<220>
<223>  Core sequence


<220>
<221>  VARIANT
<222>  (3)..(3)
<223>  /replace= "Leu"

<220>
<221>  VARIANT
<222>  (7)..(7)
<223>  /replace= "Val"

<400>  15

Val Lys Met Ala Leu Asp Ile Glu Ile
1               5


<210>  16
<211>  9
<212>  PRT
<213>  Artificial Sequence

<220>
<223>  iRGD peptide

<400>  16

Cys Arg Gly Asp Lys Gly Pro Asp Cys
1               5


<210>  17
<211>  8
<212>  PRT
<213>  Artificial Sequence

<220>
<223>  Sequential Lysine amino acids

<400>  17

Lys Lys Lys Lys Lys Lys Lys Lys
1               5

<210>  18
<211>  8
<212>  PRT
<213>  Artificial Sequence

<220>
<223>  Sequential Arginine amino acids

<400>  18

Arg Arg Arg Arg Arg Arg Arg Arg
1               5


<210>  19
<211>  20
<212>  PRT
<213>  Artificial Sequence

<220>
<223>  Preferred sequence - Peptide 78 - DIF-P

<400>  19

Leu Leu Asn Val Lys Met Ala Leu Asp Ile Glu Ile Ala Thr Tyr Arg
1               5                   10                  15


Lys Leu Leu Glu
            20


<210>  20
<211>  15
<212>  PRT
<213>  Artificial Sequence

<220>
<223>  Preferred sequence - Peptide 78-12 - DIF-P3K

<400>  20

Leu Leu Asn Val Lys Met Ala Leu Asp Ile Glu Ile Lys Lys Lys
1               5                   10                  15


<210>  21
<211>  20
<212>  PRT
<213>  Artificial Sequence

<220>
<223>  Preferred sequence

<400>  21

Leu Leu Asn Val Lys Met Ala Leu Asp Val Glu Ile Ala Thr Tyr Arg
1               5                   10                  15


Lys Leu Leu Glu
            20

<210>  22
<211>  20
<212>  PRT
<213>  Artificial Sequence

<220>
<223>  Preferred sequence

<400>  22

Leu Leu Asn Val Lys Leu Ala Leu Asp Ile Glu Ile Ala Thr Tyr Arg
1               5                   10                  15

Lys Leu Leu Glu
            20

<210>  23
<211>  20
<212>  PRT
<213>  Artificial Sequence

<220>
<223>  Preferred sequence

<400>  23

Leu Leu Asn Val Lys Met Ala Leu Asp Ile Glu Ile Ala Ala Tyr Arg
1               5                   10                  15

Lys Leu Leu Glu
            20

<210>  24
<211>  20
<212>  PRT
<213>  Artificial Sequence

<220>
<223>  Preferred sequence - Peptide 77

<400>  24

Arg Glu Tyr Gln Glu Leu Leu Asn Val Lys Met Ala Leu Asp Ile Glu
1               5                   10                  15

Ile Ala Thr Tyr
            20

<210>  25
<211>  16
<212>  PRT
<213>  Artificial Sequence

<220>
<223>  Preferred sequence - Peptide 78-16

<400>  25

Leu Leu Asn Val Lys Met Ala Leu Asp Ile Glu Ile Ala Thr Tyr Arg
1               5                   10                  15


<210>  26
<211>  18
<212>  PRT
<213>  Artificial Sequence

<220>
<223>  Preferred sequence - Peptide 78-15

<400>  26

Leu Leu Asn Val Lys Met Ala Leu Asp Ile Glu Ile Ala Thr Tyr Lys
1               5                   10                  15


Lys Lys




<210>  27
<211>  17
<212>  PRT
<213>  Artificial Sequence

<220>
<223>  Preferred sequence - Peptide 78-14

<400>  27

Leu Leu Asn Val Lys Met Ala Leu Asp Ile Glu Ile Ala Thr Lys Lys
1               5                   10                  15


Lys




<210>  28
<211>  16
<212>  PRT
<213>  Artificial Sequence

<220>
<223>  Preferred sequence - Peptide 78-13

<400>  28

Leu Leu Asn Val Lys Met Ala Leu Asp Ile Glu Ile Ala Lys Lys Lys
1               5                   10                  15


<210>  29
<211>  18
<212>  PRT
<213>  Artificial Sequence

<220>

<223>  Preferred sequence - Peptide 78-A

<400>  29

Leu Asn Val Lys Met Ala Leu Asp Ile Glu Ile Ala Thr Tyr Arg Lys
1               5                   10                  15

Lys Lys

<210>  30
<211>  14
<212>  PRT
<213>  Artificial Sequence

<220>
<223>  Preferred sequence - Peptide 78-B

<400>  30

Asn Val Lys Met Ala Leu Asp Ile Glu Ile Ala Thr Tyr Arg
1               5                   10

<210>  31
<211>  13
<212>  PRT
<213>  Artificial Sequence

<220>
<223>  Preferred sequence - Peptide 78-C

<400>  31

Val Lys Met Ala Leu Asp Ile Glu Ile Ala Thr Tyr Arg
1               5                   10

<210>  32
<211>  17
<212>  PRT
<213>  Artificial Sequence

<220>
<223>  Preferred sequence

<400>  32

Leu Leu Asn Val Lys Met Ala Leu Asp Val Glu Ile Ala Thr Lys Lys
1               5                   10                  15

Lys

<210>  33
<211>  15
<212>  PRT
<213>  Artificial Sequence

<220>
<223>  Preferred sequence

<400>  33

Leu Leu Asn Val Lys Met Ala Leu Asp Val Glu Ile Lys Lys Lys
1               5                   10                  15


<210>  34
<211>  16
<212>  PRT
<213>  Artificial Sequence

<220>
<223>  Preferred sequence

<400>  34

Leu Leu Asn Val Lys Met Ala Leu Asp Val Glu Ile Ala Lys Lys Lys
1               5                   10                  15


<210>  35
<211>  15
<212>  PRT
<213>  Artificial Sequence

<220>
<223>  Preferred sequence

<400>  35

Leu Leu Asn Val Lys Leu Ala Leu Asp Ile Glu Ile Lys Lys Lys
1               5                   10                  15


<210>  36
<211>  16
<212>  PRT
<213>  Artificial Sequence

<220>
<223>  Preferred sequence

<400>  36

Leu Leu Asn Val Lys Leu Ala Leu Asp Ile Glu Ile Ala Lys Lys Lys
1               5                   10                  15


<210>  37
<211>  17
<212>  PRT
<213>  Artificial Sequence

<220>
<223>  Preferred sequence

<400>  37

Leu Leu Asn Val Lys Leu Ala Leu Asp Ile Glu Ile Ala Thr Lys Lys
1               5                   10                  15

Lys

<210>  38
<211>  15
<212>  PRT
<213>  Artificial Sequence

<220>
<223>  Preferred sequence

<400>  38

Leu Leu Asn Val Lys Leu Ala Leu Asp Val Glu Ile Lys Lys Lys
1               5                   10                  15

<210>  39
<211>  20
<212>  PRT
<213>  Artificial Sequence

<220>
<223>  Preferred sequence

<400>  39

Leu Leu Asn Val Lys Met Ala Leu Asp Ile Glu Ile Arg Arg Arg Arg
1               5                   10                  15

Arg Arg Arg Arg
            20

<210>  40
<211>  20
<212>  PRT
<213>  Artificial Sequence

<220>
<223>  Peptide 76

<400>  40

Met Ala Arg His Leu Arg Glu Tyr Gln Glu Leu Leu Asn Val Lys Met
1               5                   10                  15

Ala Leu Asp Ile
            20

<210>  41
<211>  12
<212>  PRT
<213>  Artificial Sequence

<220>
<223>  Peptide 78-D

<400>  41

Lys Met Ala Leu Asp Ile Glu Ile Ala Thr Tyr Arg
1               5                   10


<210>  42
<211>  20
<212>  PRT
<213>  Artificial Sequence

<220>
<223>  Peptide 79

<400>  42

Met Ala Leu Asp Ile Glu Ile Ala Thr Tyr Arg Lys Leu Leu Glu Gly
1               5                   10                  15


Glu Glu Ser Arg
            20


<210>  43
<211>  9
<212>  PRT
<213>  Artificial Sequence

<220>
<223>  Variant of core sequence

<400>  43

Ala Lys Leu Gly Leu Asp Ile Glu Ile
1               5


<210>  44
<211>  9
<212>  PRT
<213>  Artificial Sequence

<220>
<223>  Variant of core sequence

<400>  44

Ile Lys Leu Ala Leu Asp Val Glu Ile
1               5


<210>  45
<211>  9
<212>  PRT
<213>  Artificial Sequence

<220>
<223>  Variant of core sequence

<400>  45

Ile Lys Leu Ala Leu Asp Met Glu Ile
1               5

<210>  46
<211>  9
<212>  PRT
<213>  Artificial Sequence

<220>
<223>  Variant of core sequence

<400>  46

Leu Lys Leu Ala Leu Asp Met Glu Ile
1               5

<210>  47
<211>  9
<212>  PRT
<213>  Artificial Sequence

<220>
<223>  Variant of core sequence

<400>  47

Ser Lys Leu Gly Leu Asp Ile Glu Ile
1               5

<210>  48
<211>  9
<212>  PRT
<213>  Artificial Sequence

<220>
<223>  Variant of core sequence

<400>  48

Thr Lys Leu Ala Leu Asp Leu Glu Ile
1               5

<210>  49
<211>  9
<212>  PRT
<213>  Artificial Sequence

<220>
<223>  Variant of core sequence

<400>  49

Thr Lys Leu Ala Leu Asp Val Glu Ile
1               5

<210>  50
<211>  9
<212>  PRT

<213>  Artificial Sequence

<220>
<223>  Variant of core sequence

<400>  50

Thr Lys Leu Ser Leu Asp Val Glu Ile
1               5


<210>  51
<211>  9
<212>  PRT
<213>  Artificial Sequence

<220>
<223>  Variant of core sequence - GFAP9

<400>  51

Val Lys Leu Ala Leu Asp Ile Glu Ile
1               5


<210>  52
<211>  9
<212>  PRT
<213>  Artificial Sequence

<220>
<223>  Variant of core sequence

<400>  52

Val Lys Leu Ala Leu Asp Met Glu Ile
1               5


<210>  53
<211>  9
<212>  PRT
<213>  Artificial Sequence

<220>
<223>  Variant of core sequence

<400>  53

Val Lys Leu Ala Leu Asp Val Glu Ile
1               5


<210>  54
<211>  9
<212>  PRT
<213>  Artificial Sequence

<220>
<223>  Variant of core sequence

<400>  54

Val Lys Leu Ser Leu Asp Ile Glu Ile
1               5


<210>  55
<211>  9
<212>  PRT
<213>  Artificial Sequence

<220>
<223>  Variant of core sequence - Per9

<400>  55

Val Lys Met Ala Leu Asp Ile Glu Ile
1               5


<210>  56
<211>  9
<212>  PRT
<213>  Artificial Sequence

<220>
<223>  Variant of core sequence - Des9

<400>  56

Val Lys Met Ala Leu Asp Val Glu Ile
1               5


<210>  57
<211>  9
<212>  PRT
<213>  Artificial Sequence

<220>
<223>  Variant of core sequence

<400>  57

Val Lys Ser Arg Leu Glu Cys Glu Ile
1               5


<210>  58
<211>  9
<212>  PRT
<213>  Artificial Sequence

<220>
<223>  Variant of core sequence

<400>  58

Val Lys Thr Arg Leu Glu Asn Glu Ile
1               5


<210>  59
<211>  9
<212>  PRT

<213>  Artificial Sequence

<220>
<223>  Variant of core sequence

<400>  59

Val Lys Thr Arg Leu Glu Gln Glu Ile
1               5


<210>  60
<211>  9
<212>  PRT
<213>  Artificial Sequence

<220>
<223>  Variant of core sequence

<400>  60

Val Arg Ala Arg Leu Glu Cys Glu Ile
1               5


<210>  61
<211>  9
<212>  PRT
<213>  Artificial Sequence

<220>
<223>  Variant of core sequence

<400>  61

Val Arg Ala Arg Leu Glu Gly Glu Ile
1               5


<210>  62
<211>  9
<212>  PRT
<213>  Artificial Sequence

<220>
<223>  Variant of core sequence

<400>  62

Val Lys Ala Arg Leu Glu Gly Glu Ile
1               5


<210>  63
<211>  9
<212>  PRT
<213>  Artificial Sequence

<220>
<223>  Variant of core sequence

<400>  63

Val Lys Ala Arg Leu Glu Leu Glu Ile
1               5


<210>  64
<211>  9
<212>  PRT
<213>  Artificial Sequence

<220>
<223>  Variant of core sequence

<400>  64

Val Lys Ala Arg Leu Glu Asn Glu Ile
1               5


<210>  65
<211>  9
<212>  PRT
<213>  Artificial Sequence

<220>
<223>  Variant of core sequence

<400>  65

Val Lys Ile Phe Leu Glu Lys Glu Ile
1               5


<210>  66
<211>  9
<212>  PRT
<213>  Artificial Sequence

<220>
<223>  Variant of core sequence

<400>  66

Ile Lys Thr Arg Leu Glu Gln Glu Ile
1               5


<210>  67
<211>  9
<212>  PRT
<213>  Artificial Sequence

<220>
<223>  Variant of core sequence

<400>  67

Ile Lys Ile Arg Leu Glu Asn Glu Ile
1               5


<210>  68
<211>  9
<212>  PRT

<213>  Artificial Sequence

<220>
<223>  Variant of core sequence

<400>  68

Ile Lys Thr Arg Leu Glu Val Glu Ile
1               5


<210>  69
<211>  9
<212>  PRT
<213>  Artificial Sequence

<220>
<223>  Variant of core sequence

<400>  69

Ile Lys Val Lys Leu Glu Ala Glu Ile
1               5


<210>  70
<211>  9
<212>  PRT
<213>  Artificial Sequence

<220>
<223>  Variant of core sequence

<400>  70

Ile Lys Ser Arg Leu Glu Gln Glu Ile
1               5


<210>  71
<211>  9
<212>  PRT
<213>  Artificial Sequence

<220>
<223>  Variant of core sequence

<400>  71

Val Lys Val His Leu Glu Lys Glu Ile
1               5


<210>  72
<211>  9
<212>  PRT
<213>  Artificial Sequence

<220>
<223>  Variant of core sequence

<400>  72

Ile Lys Leu His Leu Glu Lys Glu Ile
1               5

<210>  73
<211>  9
<212>  PRT
<213>  Artificial Sequence

<220>
<223>  Variant of core sequence

<400>  73

Ile Lys Thr His Leu Glu Lys Glu Ile
1               5

<210>  74
<211>  9
<212>  PRT
<213>  Artificial Sequence

<220>
<223>  Variant of core sequence

<400>  74

Ile Lys Met Arg Leu Glu Lys Glu Ile
1               5

<210>  75
<211>  20
<212>  PRT
<213>  Artificial Sequence

<220>
<223>  Peptide 72

<400>  75

Gly Gly Tyr Gln Ala Gly Ala Ala Arg Leu Glu Glu Glu Leu Arg Gln
1               5                   10                  15

Leu Lys Glu Glu
            20

<210>  76
<211>  28
<212>  PRT
<213>  Artificial Sequence

<220>
<223>  Peptide 72-8R

<400>  76

Gly Gly Tyr Gln Ala Gly Ala Ala Arg Leu Glu Glu Glu Leu Arg Gln
1               5                   10                  15

Leu Lys Glu Glu Arg Arg Arg Arg Arg Arg Arg Arg
            20                  25

<210>  77
<211>  20
<212>  PRT
<213>  Artificial Sequence

<220>
<223>  LMNA

<400>  77

Leu Leu Asp Ile Lys Leu Ala Leu Asp Met Glu Ile His Ala Tyr Arg
1               5                   10                  15

Lys Leu Leu Glu
            20

<210>  78
<211>  20
<212>  PRT
<213>  Artificial Sequence

<220>
<223>  KRT84

<400>  78

Leu Met Asn Ala Lys Leu Gly Leu Asp Ile Glu Ile Ala Thr Tyr Arg
1               5                   10                  15

Arg Leu Leu Glu
            20

<210>  79
<211>  20
<212>  PRT
<213>  Artificial Sequence

<220>
<223>  KRT32

<400>  79

Leu Leu Asp Val Arg Ala Arg Leu Glu Gly Glu Ile Asn Thr Tyr Arg
1               5                   10                  15

Ser Leu Leu Glu
            20

<210>  80
<211>  20
<212>  PRT
<213>  Artificial Sequence

<220>
<223>  KRT13

<400>  80

Leu Leu Asp Ile Lys Thr Arg Leu Glu Gln Glu Ile Ala Thr Tyr Arg
1               5                   10                  15

Ser Leu Leu Glu
            20

<210>  81
<211>  20
<212>  PRT
<213>  Artificial Sequence

<220>
<223>  GFAP

<400>  81

Leu Leu Asn Val Lys Leu Ala Leu Asp Ile Glu Ile Ala Thr Tyr Arg
1               5                   10                  15

Lys Leu Leu Glu
            20

<210>  82
<211>  20
<212>  PRT
<213>  Artificial Sequence

<220>
<223>  NF

<400>  82

Leu Leu Asn Val Lys Met Ala Leu Asp Ile Glu Ile Ala Ala Tyr Arg
1               5                   10                  15

Lys Leu Leu Glu
            20

<210>  83
<211>  20
<212>  PRT
<213>  Artificial Sequence

<220>
<223>  DES

<400>  83

Leu Leu Asn Val Lys Met Ala Leu Asp Val Glu Ile Ala Thr Tyr Arg
1               5                   10                  15

Lys Leu Leu Glu
            20

<210>  84
<211>  20
<212>  PRT
<213>  Artificial Sequence

<220>
<223>  KRT18-8R

<400>  84

Leu Leu Asn Ile Lys Val Lys Leu Glu Ala Glu Ile Arg Arg Arg Arg
1               5                   10                  15

Arg Arg Arg Arg
            20

<210>  85
<211>  20
<212>  PRT
<213>  Artificial Sequence

<220>
<223>  KRT17-8R

<400>  85

Leu Leu Asp Val Lys Thr Arg Leu Glu Gln Glu Ile Arg Arg Arg Arg
1               5                   10                  15

Arg Arg Arg Arg
            20

<210>  86
<211>  20
<212>  PRT
<213>  Artificial Sequence

<220>
<223>  DES-8R

<400>  86

Leu Leu Asn Val Lys Met Ala Leu Asp Val Glu Ile Arg Arg Arg Arg
1               5                   10                  15

Arg Arg Arg Arg
            20